# EUROPEAN PATENT APPLICATION

(11) **EP 1 992 640 A1**
(43) Date of publication of application: **19.11.2008**
(21) Application number: 08012753.3
(22) Date of filing: 22.12.1998
(51) Int. Cl.: C07K 14/47, C12N 15/12, C12Q 1/68, G01N 33/574, C07K 16/30, A61K 48/00, C12N 5/08

(54) **Compounds for immunotherapy and diagnosis of breast cancer and methods for their use**

(30) Priority: 24.12.1997 US 998253; 24.12.1997 US 998255; 17.07.1998 US 118627; 17.07.1998 US 118554
(62) Divisional of application: 98964884.5
(71) Applicant: CORIXA CORPORATION, Wilmington, DE 19808 (US)
(72) Inventor: The designation of the inventor has not yet been filed
(74) Representative: Thornton, Neil

(57) **Abstract**

Compounds and methods for the treatment and diagnosis of breast cancer are provided. The inventive compounds include polypeptides containing at least a portion of a breast tumor protein. Vaccines and pharmaceutical compositions for immunotherapy of breast cancer comprising such polypeptides, or polynucleotide molecules encoding such polypeptides, are also provided, together with polynucleotide molecules for preparing the inventive polypeptides.

## Description

### TECHNICAL FIELD

The present invention relates generally to compositions and methods for the treatment and diagnosis of breast cancer. The invention is more particularly related to polypeptides comprising at least a portion of a protein that is preferentially expressed in breast tumor tissue and to polynucleotide molecules encoding such polypeptides. Such polypeptides may be used in vaccines and pharmaceutical compositions for treatment of breast cancer. Additionally such polypeptides and polynucleotides may be used in the immunodiagnosis of breast cancer.

### BACKGROUND OF THE INVENTION

Breast cancer is a significant health problem for women in the United States and throughout the world. Although advances have been made in detection and treatment of the disease, breast cancer remains the second leading cause of cancer-related deaths in women, affecting more than 180,000 women in the United States each year. For women in North America, the life-time odds of getting breast cancer are now one in eight.

No vaccine or other universally successful method for the prevention or treatment of breast cancer is currently available. Management of the disease currently relies on a combination of early diagnosis (through routine breast screening procedures) and aggressive treatment, which may include one or more of a variety of treatments such as surgery, radiotherapy, chemotherapy and hormone therapy. The course of treatment for a particular breast cancer is often selected based on a variety of prognostic parameters, including an analysis of specific tumor markers. *See, e.g.,* Porter-Jordan and Lippman, Breast Cancer 8:73-100 (1994). However, the use of established markers often leads to a result that is difficult to interpret, and the high mortality observed in breast cancer patients indicates that improvements are needed in the treatment, diagnosis and prevention of the disease.

Accordingly, there is a need in the art for improved methods for therapy and diagnosis of breast cancer. The present invention fulfills these needs and further provides other related advantages.

### SUMMARY OF THE INVENTION

The present invention provides compounds and methods for immunotherapy of breast cancer. In one aspect, isolated polypeptides are provided comprising at least an immunogenic portion of a breast tumor protein or a variant of said protein that differs only in conservative substitutions and/or modifications, wherein the breast tumor protein comprises an amino acid sequence encoded by a polynucleotide molecule having a partial sequence selected from the group consisting of (a) nucleotide sequences recited in SEQ ID NOS: 3, 10, 17, 24, 45-52 and 55-67, 72, 73, and 89-94, (b) complements of said nucleotide sequences and (c) sequences that hybridize to a sequence of (a) or (b) under moderately stringent conditions.

In related aspects, isolated polynucleotide molecules encoding the above polypeptides are provided. In specific embodiments, such polynucleotide molecules have partial sequences provided in SEQ ID NOS: 3, 10, 17, 24, 45-52 and 55-67, 72, 73, and 89-94. The present invention further provides expression vectors comprising the above polynucleotide molecules and host cells transformed or transfected with such expression vectors. In preferred embodiments, the host cells are selected from the group consisting of *E*. *coli,* yeast and mammalian cells.

In another aspect, the present invention provides fusion proteins comprising a first and a second inventive polypeptide or, alternatively, an inventive polypeptide and a known breast antigen.

The present invention also provides pharmaceutical compositions comprising at least one of the above polypeptides, or a polynucleotide molecule encoding such a polypeptide, and a physiologically acceptable carrier, together with vaccines comprising at least one or more such polypeptide or polynucleotide molecule in combination with a non-specific immune response enhancer. Pharmaceutical compositions and vaccines comprising one or more of the above fusion proteins are also provided.

In related aspects, pharmaceutical compositions for the treatment of breast cancer comprising at least one polypeptides and a physiologically acceptable carrier are provided, wherein the polypeptide comprises an immunogenic portion of a breast tumor protein or a variant thereof, the breast tumor protein being encoded by a polynucleotide molecule having a partial sequence selected from the group consisting of: (a) nucleotide sequences recited in SEQ ID NOS: 1, 2, 4-9, 11-16, 18-23, 25-44, 53, 54, 68-71, and 74-88, (b) complements of said nucleotide sequences, and (c) sequences that hybridize to a sequence of (a) or (b) under moderately stringent conditions. The invention also provides vaccines for the treatment of breast cancer comprising such polypeptides in combination with a non-specific immune response enhancer, together with pharmaceutical compositions and vaccines comprising at least one polynucleotide molecule having a partial sequence provided in SEQ ID NOS: 1, 2, 4-9, 11-16, 18-23, 25-44, 53, 54, 68-71, and 74-88.

In yet another aspect, methods are provided for inhibiting the development of breast cancer in a patient, comprising administering an effective amount of at least one of the above pharmaceutical compositions and/or vaccines.

The present invention also provides methods for immunodiagnosis of breast cancer, together with kits for use in such methods. In one specific aspect of the present invention, methods are provided for detecting breast cancer in a patient, comprising: (a) contacting a biological sample obtained from a patient with a binding agent that is capable of binding to one of the inventive polypeptides; and (b) detecting in the sample a protein or polypeptide that binds to the binding agent. In preferred embodiments, the binding agent is an antibody, most preferably a monoclonal antibody.

In related aspects, methods are provided for monitoring the progression of breast cancer in a patient, comprising: (a) contacting a biological sample obtained from a patient with a binding agent that is capable of binding to one of the above polypeptides; (b) determining in the sample an amount of a protein or polypeptide that binds to the binding agent; (c) repeating steps (a) and (b); and comparing the amounts of polypeptide detected in steps (b) and (c).

Within related aspects, the present invention provides antibodies, preferably monoclonal antibodies, that bind to the inventive polypeptides, as well as diagnostic kits comprising such antibodies, and methods of using such antibodies to inhibit the development of breast cancer.

The present invention further provides methods for detecting breast cancer comprising: (a) obtaining a biological sample from a patient; (b) contacting the sample with a first and a second oligonucleotide primer in a polymerase chain reaction, at least one of the oligonucleotide primers being specific for a DNA molecule that encodes one of the above polypeptides; and (c) detecting in the sample a DNA sequence that amplifies in the presence of the first and second oligonucleotide primers. In a preferred embodiment, at least one of the oligonucleotide primers comprises at least about 10 contiguous nucleotides of a DNA molecule having a partial sequence selected from the group consisting of SEQ ID NOS: 1-94.

In a further aspect, the present invention provides a method for detecting breast cancer in a patient comprising: (a) obtaining a biological sample from the patient; (b) contacting the sample with an oligonucleotide probe specific for a polynucleotide molecule that encodes one of the above polypeptides; and (c) detecting in the sample a polynucleotide sequence that hybridizes to the oligonucleotide probe. Preferably, the oligonucleotide probe comprises at least about 15 contiguous nucleotides of a DNA molecule having a partial sequence selected from the group consisting of SEQ ID NOS: 1-94.

In related aspects, diagnostic kits comprising the above oligonucleotide probes or primers are provided.

These and other aspects of the present invention will become apparent upon reference to the following detailed description. All references disclosed herein are hereby incorporated by reference in their entirety as if each was incorporated individually.

### BRIEF DESCRIPTION OF THE DRAWINGS

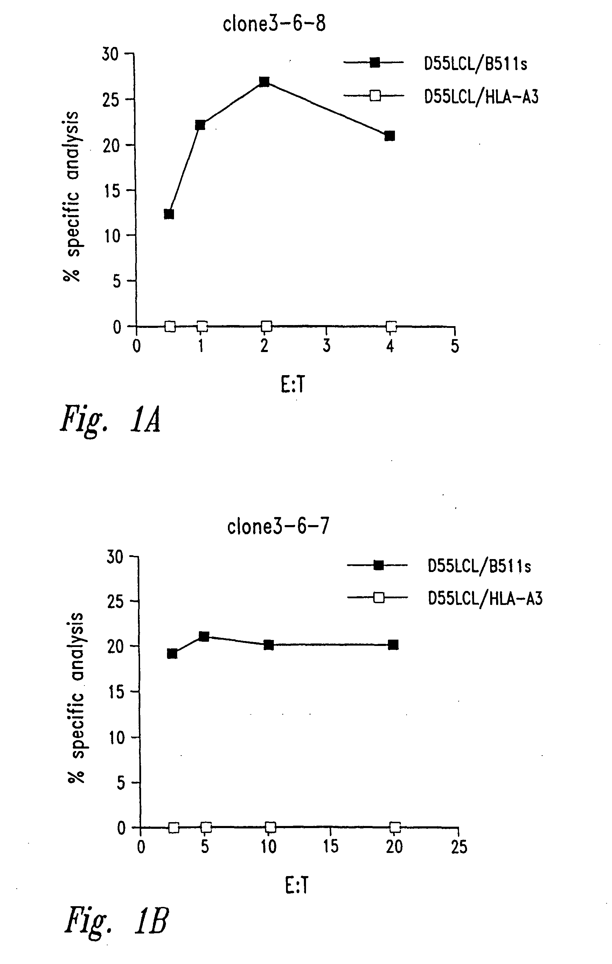

Figs. 1A and B show the specific lytic activity of a first and a second B511S-specific CTL clone, respectively, measured on autologous LCL transduced with B511s (filled squares) or HLA-A3 (open squares).

### DETAILED DESCRIPTION OF THE INVENTION

As noted above, the present invention is generally directed to compositions and methods for the immunotherapy and diagnosis of breast cancer. The inventive compositions are generally isolated polypeptides that comprise at least a portion of a breast tumor protein. Also included within the present invention are molecules (such as an antibody or fragment thereof) that bind to the inventive polypeptides. Such molecules are referred to herein as "binding agents."

In particular, the subject invention discloses polypeptides comprising at least a portion of a human breast tumor protein, or a variant thereof, wherein the breast tumor protein includes an amino acid sequence encoded by a polynucleotide molecule including a sequence selected from the group consisting of: nucleotide sequences recited in SEQ ID NOS: 1- 94, the complements of said nucleotide sequences, and variants thereof. As used herein, the term "polypeptide" encompasses amino acid chains of any length, including full length proteins, wherein the amino acid residues are linked by covalent peptide bonds. Thus, a polypeptide comprising a portion of one of the above breast proteins may consist entirely of the portion, or the portion may be present within a larger polypeptide that contains additional sequences. The additional sequences may be derived from the native protein or may be heterologous, and such sequences may be immunoreactive and/or antigenic.

As used herein, an "immunogenic portion" of a human breast tumor protein is a portion that is capable of eliciting an immune response in a patient inflicted with breast cancer and as such binds to antibodies present within sera from a breast cancer patient. Such immunogenic portions generally comprise at least about 5 amino acid residues, more preferably at least about 10, and most preferably at least about 20 amino acid residues. Immunogenic portions of the proteins described herein may be identified in antibody binding assays. Such assays may generally be performed using any of a variety of means known to those of ordinary skill in the art, as described, for example, in Harlow and Lane, Antibodies: A Laboratory Manual, Cold Spring Harbor Laboratory, Cold Spring Harbor, NY, 1988. For example, a polypeptide may be immobilized on a solid support (as described below) and contacted with patient sera to allow binding of antibodies within the sera to the immobilized polypeptide. Unbound sera may then be removed and bound antibodies detected using, for example, ¹²⁵I-labeled Protein A. Alternatively, a polypeptide may be used to generate monoclonal and polyclonal antibodies for use in detection of the polypeptide in blood or other fluids of breast cancer patients. Methods for preparing and identifying immunogenic portions of antigens of known sequence are well known in the art and include those summarized in Paul, Fundamental Immunology, 3rd ed., Raven Press, 1993, pp. 243-247.

The term "polynucleotide(s)," as used herein, means a single or double-stranded polymer of deoxyribonucleotide or ribonucleotide bases and includes DNA and corresponding RNA molecules, including HnRNA and mRNA molecules, both sense and anti-sense strands, and comprehends cDNA, genomic DNA and recombinant DNA, as well as wholly or partially synthesized polynucleotides. An HnRNA molecule contains introns and corresponds to a DNA molecule in a generally one-to-one manner. An mRNA molecule corresponds to an HnRNA and DNA molecule from which the introns have been excised. A polynucleotide may consist of an entire gene, or any portion thereof. Operable anti-sense polynucleotides may comprise a fragment of the corresponding polynucleotide, and the definition of "polynucleotide" therefore includes all such operable anti-sense fragments.

The compositions and methods of the present invention also encompass variants of the above polypeptides and polynucleotides. A polypeptide "variant," as used herein, is a polypeptide that differs from the recited polypeptide only in conservative substitutions and/or modifications, such that the therapeutic, antigenic and/or immunogenic properties of the polypeptide are retained. In a preferred embodiment, variant polypeptides differ from an identified sequence by substitution, deletion or addition of five amino acids or fewer. Such variants may generally be identified by modifying one of the above polypeptide sequences, and evaluating the antigenic properties of the modified polypeptide using, for example, the representative procedures described herein. Polypeptide variants preferably exhibit at least about 70%, more preferably at least about 90% and most preferably at least about 95% identity (determined as described below) to the identified polypeptides.

As used herein, a "conservative substitution" is one in which an amino acid is substituted for another amino acid that has similar properties, such that one skilled in the art of peptide chemistry would expect the secondary structure and hydropathic nature of the polypeptide to be substantially unchanged. In general, the following groups of amino acids represent conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

Variants may also, or alternatively, contain other modifications, including the deletion or addition of amino acids that have minimal influence on the antigenic properties, secondary structure and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification or identification of the polypeptide (e.g., poly-His), or to enhance binding of the polypeptide to a solid support. For example, a polypeptide may be conjugated to an immunoglobulin Fc region.

A nucleotide "variant" is a sequence that differs from the recited nucleotide sequence in having one or more nucleotide deletions, substitutions or additions. Such modifications may be readily introduced using standard mutagenesis techniques, such as oligonucleotide-directed site-specific mutagenesis as taught, for example, by Adelman et al. (DNA, 2:183, 1983). Nucleotide variants may be naturally occurring allelic variants, or non-naturally occurring variants. Variant nucleotide sequences preferably exhibit at least about 70%, more preferably at least about 80% and most preferably at least about 90% identity (determined as described below) to the recited sequence.

The antigens provided by the present invention include variants that are encoded by DNA sequences which are substantially homologous to one or more of the DNA sequences specifically recited herein. "Substantial homology," as used herein, refers to DNA sequences that are capable of hybridizing under moderately stringent conditions. Suitable moderately stringent conditions include prewashing in a solution of 5X SSC, 0.5% SDS, 1.0 mM EDTA (pH 8.0); hybridizing at 50°C-65°C, 5X SSC, overnight or, in the event of cross-species homology, at 45°C with 0.5X SSC; followed by washing twice at 65°C for 20 minutes with each of 2X, 0.5X and 0.2X SSC containing 0.1 % SDS. Such hybridizing DNA sequences are also within the scope of this invention, as are nucleotide sequences that, due to code degeneracy, encode an immunogenic polypeptide that is encoded by a hybridizing DNA sequence.

Two nucleotide or polypeptide sequences are said to be "identical" if the sequence of nucleotides or amino acid residues in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, more preferably 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Resarch Foundaiton, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) Fast and sensitive multiple sequence alignments on a microcomputer CABIOS 5:151-153; Myers, E.W. and Muller W. (1988) Optimal alignments in linear space CABIOS 4:11-17; Robinson, E.D. (1971) Comb. Theor 11:105; Santou, N. Nes, M. (1987) The neighbor joining method. A new method for reconstructing phylogenetic trees Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Rapid similarity searches of nucleic acid and protein data banks Proc. Natl. Acad., Sci. USA 80:726-730.

Preferably, the "percentage of sequence identity" is determined by comparing two optimally aligned sequences over a window of comparison of at least 20 positions, wherein the portion of the polynucleotide sequence in the comparison window may comprise additions or deletions (i.e. gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i.e. the window size) and multiplying the results by 100 to yield the percentage of sequence identity.

Also included in the scope of the present invention are alleles of the genes encoding the nucleotide sequences recited herein. As used herein, an "allele" or "allellic sequence" is an alternative form of the gene which may result from at least one mutation in the nucleic acid sequence. Alleles may result in altered mRNAs or polypeptides whose structure or function may or may not be altered. Any given gene may have none, one, or many allelic forms. Common mutational changes which give rise to alleles are generally ascribed to natural deletions, additions, or substitutions of nucleotides. Each of these types of changes may occur alone or in combination with the others, one or more times in a given sequence.

For breast tumor polypeptides with immunoreactive properties, variants may, alternatively, be identified by modifying the amino acid sequence of one of the above polypeptides, and evaluating the immunoreactivity of the modified polypeptide. For breast tumor polypeptides useful for the generation of diagnostic binding agents, a variant may be identified by evaluating a modified polypeptide for the ability to generate antibodies that detect the presence or absence of breast cancer. Such modified sequences may be prepared and tested using, for example, the representative procedures described herein.

The breast tumor proteins of the present invention, and polynucleotide molecules encoding such proteins, may be isolated from breast tumor tissue using any of a variety of methods well known in the art. Polynucleotide sequences corresponding to a gene (or a portion thereof) encoding one of the inventive breast tumor proteins may be isolated from a breast tumor cDNA library using a subtraction technique as described in detail below. Examples of such DNA sequences are provided in SEQ ID NOS: 1- 94. Partial polynucleotide sequences thus obtained may be used to design oligonucleotide primers for the amplification of full-length polynucleotide sequences in a polymerase chain reaction (PCR), using techniques well known in the art (see, for example, Mullis et al., Cold Spring Harbor Symp. Quant. Biol., 51:263, 1987; Erlich ed., PCR Technology, Stockton Press, NY, 1989). Once a polynucleotide sequence encoding a polypeptide is obtained, any of the above modifications may be readily introduced using standard mutagenesis techniques, such as oligonucleotide-directed site-specific mutagenesis as taught, for example, by Adelman et al. (DNA, 2:183, 1983).

The breast tumor polypeptides disclosed herein may also be generated by synthetic or recombinant means. Synthetic polypeptides having fewer than about 100 amino acids, and generally fewer than about 50 amino acids, may be generated using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain (see, for example, Merrifield, J. Am. Chem. Soc. 85:2149-2146, 1963). Equipment for automated synthesis of polypeptides is commercially available from suppliers such as Perkin Elmer/Applied BioSystems Division (Foster City, CA), and may be operated according to the manufacturer's instructions.

Alternatively, any of the above polypeptides may be produced recombinantly by inserting a polynucleotide sequence that encodes the polypeptide into an expression vector and expressing the protein in an appropriate host. Any of a variety of expression vectors known to those of ordinary skill in the art may be employed to express recombinant polypeptides of this invention. Expression may be achieved in any appropriate host cell that has been transformed or transfected with an expression vector containing a polynucleotide molecule that encodes a recombinant polypeptide. Suitable host cells include prokaryotes, yeast and higher eukaryotic cells. Preferably, the host cells employed are *E. coli,* yeast or a mammalian cell line, such as CHO cells. The polynucleotide sequences expressed in this manner may encode naturally occurring polypeptides, portions of naturally occurring polypeptides, or other variants thereof.

In general, regardless of the method of preparation, the polypeptides disclosed herein are prepared in an isolated, substantially pure form *(i.e.,* the polypeptides are homogenous as determined by amino acid composition and primary sequence analysis). Preferably, the polypeptides are at least about 90% pure, more preferably at least about 95% pure and most preferably at least about 99% pure. In certain preferred embodiments, described in more detail below, the substantially pure polypeptides are incorporated into pharmaceutical compositions or vaccines for use in one or more of the methods disclosed herein.

In a related aspect, the present invention provides fusion proteins comprising a first and a second inventive polypeptide or, alternatively, a polypeptide of the present invention and a known breast tumor antigen, together with variants of such fusion proteins.

A polynucleotide sequence encoding a fusion protein of the present invention is constructed using known recombinant DNA techniques to assemble separate polynucleotide sequences encoding the first and second polypeptides into an appropriate expression vector. The 3' end of a polynucleotide sequence encoding the first polypeptide is ligated, with or without a peptide linker, to the 5' end of a polynucleotide sequence encoding the second polypeptide so that the reading frames of the sequences are in phase to permit mRNA translation of the two DNA sequences into a single fusion protein that retains the biological activity of both the first and the second polypeptides.

A peptide linker sequence may be employed to separate the first and the second polypeptides by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may be from 1 to about 50 amino acids in length. Peptide sequences are not required when the first and second polypeptides have non-essential N-terminal amino acid regions that can be used to separate the functional domains and prevent steric interference.

The ligated polynucleotide sequences are operably linked to suitable transcriptional or translational regulatory elements. The regulatory elements responsible for expression of polynucleotides are located only 5' to the polynucleotide sequence encoding the first polypeptides. Similarly, stop codons require to end translation and transcription termination signals are only present 3' to the polynucleotide sequence encoding the second polypeptide.

Fusion proteins are also provided that comprise a polypeptide of the present invention together with an unrelated immunogenic protein. Preferably the immunogenic protein is capable of eliciting a recall response. Examples of such proteins include tetanus, tuberculosis and hepatitis proteins (see, for example, Stoute et al. New Engl. J Med., 336:86-91 (1997)).

Polypeptides of the present invention that comprise an immunogenic portion of a breast tumor protein may generally be used for immunotherapy of breast cancer, wherein the polypeptide stimulates the patient's own immune response to breast tumor cells. In further aspects, the present invention provides methods for using one or more of the immunoreactive polypeptides encoded by a polynucleotide molecule having a sequence provided in SEQ ID NOS: 1- 94 (or fusion proteins comprising one or more such polypeptides and/or polynucleotides encoding such polypeptides) for immunotherapy of breast cancer in a patient. As used herein, a "patient" refers to any warm-blooded animal, preferably a human. A patient may be afflicted with a disease, or may be free of detectable disease. Accordingly, the above immunoreactive polypeptides (or fusion proteins or polynucleotide molecules encoding such polypeptides) may be used to treat breast cancer or to inhibit the development of breast cancer. The polypeptides may be administered either prior to or following surgical removal of primary tumors and/or treatment by administration of radiotherapy and conventional chemotherapeutic drugs.

In these aspects, the polypeptide or fusion protein is generally present within a pharmaceutical composition and/or a vaccine. Pharmaceutical compositions may comprise one or more polypeptides, each of which may contain one or more of the above sequences (or variants thereof), and a physiologically acceptable carrier. The vaccines may comprise one or more of such polypeptides and a non-specific immune response enhancer, wherein the non-specific immune response enhancer is capable of eliciting or enhancing an immune response to an exogenous antigen. Examples of non-specific-immune response enhancers include adjuvants, biodegradable microspheres (e.g., polylactic galactide) and liposomes (into which the polypeptide is incorporated). Pharmaceutical compositions and vaccines may also contain other epitopes of breast tumor antigens, either incorporated into a combination polypeptide (*i.e.*, a single polypeptide that contains multiple epitopes) or present within a separate polypeptide.

Alternatively, a pharmaceutical composition or vaccine may contain polynucleotides encoding one or more of the above polypeptides, such that the polypeptide is generated *in situ.* In such pharmaceutical compositions and vaccines, the polynucleotide may be present within any of a variety of delivery systems known to those of ordinary skill in the art, including nucleic acid expression systems, bacteria and viral expression systems. Appropriate nucleic acid expression systems contain the necessary polynucleotide sequences for expression in the patient (such as a suitable promoter). Bacterial delivery systems involve the administration of a bacterium (such as *Bacillus-Calmette-Guerrin)* that expresses an epitope of a breast tumor cell antigen on its cell surface. In a preferred embodiment, the polynucleotide molecules may be introduced using a viral expression system (e.g., vaccinia or other pox virus, retrovirus, or adenovirus), which may involve the use of a non-pathogenic (defective), replication competent virus. Suitable systems are disclosed, for example, in Fisher-Hoch et al., PNAS 86:317-321, 1989; Flexner et al., Ann. N. Y Acad. Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., PNAS 91:215-219, 1994; Kass-Eisler et al., PNAS 90:11498-11502, 1993; Guzman et al., Circulation 88:2838-2848, 1993; and Guzman et al., Cir. Res. 73:1202-1207, 1993. Techniques for incorporating polynucleotides into such expression systems are well known to those of ordinary skill in the art. The polynucleotides may also be "naked," as described, for example, in published PCT application WO 90/11092, and Ulmer et al., Science 259:1745-1749, 1993, reviewed by Cohen, Science 259:1691-1692, 1993. The uptake of naked polynucleotides may be increased by coating the polynucleotides onto biodegradable beads, which are efficiently transported into the cells.

Routes and frequency of administration, as well as dosage, will vary from individual to individual and may parallel those currently being used in immunotherapy of other diseases. In general, the pharmaceutical compositions and vaccines may be administered by injection (e.g., intracutaneous, intramuscular, intravenous or subcutaneous), intranasally (e.g., by aspiration) or orally. Between 1 and 10 doses may be administered over a 3-24 week period. Preferably, 4 doses are administered, at an interval of 3 months, and booster administrations may be given periodically thereafter. Alternate protocols may be appropriate for individual patients. A suitable dose is an amount of polypeptide or polynucleotide molecule that is effective to raise an immune response (cellular and/or humoral) against breast tumor cells in a treated patient. A suitable immune response is at least 10-50% above the basal (*i.e.,* untreated) level. In general, the amount of polypeptide present in a dose (or produced *in situ* by the polynucleotide in a dose) ranges from about 1 pg to about 100 mg per kg of host, typically from about 10 pg to about 1 mg, and preferably from about 100 pg to about 1 µg. Suitable dose sizes will vary with the size of the patient, but will typically range from about 0.01 mL to about 5 mL.

While any suitable carrier known to those of ordinary skill in the art may be employed in the pharmaceutical compositions of this invention, the type of carrier will vary depending on the mode of administration. For parenteral administration, such as subcutaneous injection, the carrier preferably comprises water, saline, alcohol, a lipid, a wax and/or a buffer. For oral administration, any of the above carriers or a solid carrier, such as mannitol, lactose, starch, magnesium stearate, sodium saccharine, talcum, cellulose, glucose, sucrose, and/or magnesium carbonate, may be employed. Biodegradable microspheres (*e.g.,* polylactic glycolide) may also be employed as carriers for the pharmaceutical compositions of this invention. Suitable biodegradable microspheres are disclosed, for example, in U.S. Patent Nos. 4,897,268 and 5,075,109.

Any of a variety of non-specific immune response enhancers may be employed in the vaccines of this invention. For example, an adjuvant may be included. Most adjuvants contain a substance designed to protect the antigen from rapid catabolism, such as aluminum hydroxide or mineral oil, and a nonspecific stimulator of immune response, such as lipid A, *Bordella pertussis* or *Mycobacterium tuberculosis.* Such adjuvants are commercially available as, for example, Freund's Incomplete Adjuvant and Complete Adjuvant (Difco Laboratories, Detroit, MI) and Merck Adjuvant 65 (Merck and Company, Inc., Rahway, NJ).

Polypeptides disclosed herein may also be employed in adoptive immunotherapy for the treatment of cancer. Adoptive immunotherapy may be broadly classified into either active or passive immunotherapy. In active immunotherapy, treatment relies on the *in vivo* stimulation of the endogenous host immune system to react against tumors with the administration of immune response-modifying agents (for example, tumor vaccines, bacterial adjuvants, and/or cytokines).

In passive immunotherapy, treatment involves the delivery of biologic reagents with established tumor-immune reactivity (such as effector cells or antibodies) that can directly or indirectly mediate antitumor effects and does not necessarily depend on an intact host immune system. Examples of effector cells include T lymphocytes (for example, CD8+ cytotoxic T-lymphocyte, CD4+ T-helper, tumor-infiltrating lymphocytes), killer cells (such as Natural Killer cells, lymphokine-activated killer cells), B cells, or antigen presenting cells (such as dendritic cells and macrophages) expressing the disclosed antigens. The polypeptides disclosed herein may also be used to generate antibodies or anti-idiotypic antibodies (as in U.S. Patent No. 4,918,164), for passive immunotherapy.

The predominant method of procuring adequate numbers of T-cells for adoptive immunotherapy is to grow immune T-cells *in vitro.* Culture conditions for expanding single antigen-specific T-cells to several billion in number with retention of antigen recognition *in vivo* are well known in the art. These *in vitro* culture conditions typically utilize intermittent stimulation with antigen, often in the presence of cytokines, such as IL-2, and non-dividing feeder cells. As noted above, the immunoreactive polypeptides described herein may be used to rapidly expand antigen-specific T cell cultures in order to generate sufficient number of cells for immunotherapy. In particular, antigen-presenting cells, such as dendritic, macrophage or B-cells, may be pulsed with immunoreactive polypeptides or transfected with a polynucleotide sequence(s), using standard techniques well known in the art. For example, antigen presenting cells may be transfected with a polynucleotide sequence, wherein said sequence contains a promoter region appropriate for increasing expression, and can be expressed as part of a recombinant virus or other expression system. For cultured T-cells to be effective in therapy, the cultured T-cells must be able to grow and distribute widely and to survive long term *in vivo.* Studies have demonstrated that cultured T-cells can be induced to grow *in vivo* and to survive long term in substantial numbers by repeated stimulation with antigen supplemented with IL-2 (see, for example, Cheever, M., et al, "Therapy With Cultured T Cells: Principles Revisited, " Immunological Reviews, 157:177, 1997).

The polypeptides disclosed herein may also be employed to generate and/or isolate tumor-reactive T-cells, which can then be administered to the patient. In one technique, antigen-specific T-cell lines may be generated by *in vivo* immunization with short peptides corresponding to immunogenic portions of the disclosed polypeptides. The resulting antigen specific CD8+ CTL clones may be isolated from the patient, expanded using standard tissue culture techniques, and returned to the patient.

Alternatively, peptides corresponding to immunogenic portions of the polypeptides may be employed to generate tumor reactive T cell subsets by selective *in vitro* stimulation and expansion of autologous T cells to provide antigen-specific T cells which may be subsequently transferred to the patient as described, for example, by Chang et al. (Crit. Rev. Oncol. Hematol., 22(3), 213, 1996). Cells of the immune system, such as T cells, may be isolated from the peripheral blood of a patient, using a commercially available cell separation system, such as CellPro Incorporated's (Bothell, WA) CEPRATE^{™} system (see U.S. Patent No. 5,240,856; U.S. Patent No. 5,215,926; WO 89/06280; WO 91/16116 and WO 92/07243). The separated cells are stimulated with one or more of the immunoreactive polypeptides contained within a delivery vehicle, such as a microsphere, to provide antigen-specific T cells. The population of tumor antigen-specific T cells is then expanded using standard techniques and the cells are administered back to the patient.

In another embodiment, T-cell and/or antibody receptors specific for the polypeptides can be cloned, expanded, and transferred into other vectors or effector cells for use in adoptive immunotherapy.

In a further embodiment, syngeneic or autologous dendritic cells may be pulsed with peptides corresponding to at least an immunogenic portion of a polypeptide disclosed herein. The resulting antigen-specific dendritic cells may either be transferred into a patient, or employed to stimulate T cells to provide antigen-specific T cells which may, in turn, be administered to a patient. The use of peptide-pulsed dendritic cells to generate antigen-specific T cells and the subsequent use of such antigen-specific T cells to eradicate tumors in a murine model has been demonstrated by Cheever et al, Immunological Reviews, 157:177, 1997).

Additionally, vectors expressing the disclosed polynucleotides may be introduced into stem cells taken from the patient and clonally propagated *in vitro* for autologous transplant back into the same patient.

Polypeptides of the present invention may also, or alternatively, be used to generate binding agents, such as antibodies or fragments thereof, that are capable of detecting metastatic human breast tumors. Binding agents of the present invention may generally be prepared using methods known to those of ordinary skill in the art, including the representative procedures described herein. Binding agents are capable of differentiating between patients with and without breast cancer, using the representative assays described herein. In other words, antibodies or other binding agents raised against a breast tumor protein, or a suitable portion thereof, will generate a signal indicating the presence of primary or metastatic breast cancer in at least about 20% of patients afflicted with the disease, and will generate a negative signal indicating the absence of the disease in at least about 90% of individuals without primary or metastatic breast cancer. Suitable portions of such breast tumor proteins are portions that are able to generate a binding agent that indicates the presence of primary or metastatic breast cancer in substantially all *(i.e.,* at least about 80%, and preferably at least about 90%) of the patients for which breast cancer would be indicated using the full length protein, and that indicate the absence of breast cancer in substantially all of those samples that would be negative when tested with full length protein. The representative assays described below, such as the two-antibody sandwich assay, may generally be employed for evaluating the ability of a binding agent to detect metastatic human breast tumors.

The ability of a polypeptide prepared as described herein to generate antibodies capable of detecting primary or metastatic human breast tumors may generally be evaluated by raising one or more antibodies against the polypeptide (using, for example, a representative method described herein) and determining the ability of such antibodies to detect such tumors in patients. This determination may be made by assaying biological samples from patients with and without primary or metastatic breast cancer for the presence of a polypeptide that binds to the generated antibodies. Such test assays may be performed, for example, using a representative procedure described below. Polypeptides that generate antibodies capable of detecting at least 20% of primary or metastatic breast tumors by such procedures are considered to be useful in assays for detecting primary or metastatic human breast tumors. Polypeptide specific antibodies may be used alone or in combination to improve sensitivity.

Polypeptides capable of detecting primary or metastatic human breast tumors may be used as markers for diagnosing breast cancer or for monitoring disease progression in patients. In one embodiment, breast cancer in a patient may be diagnosed by evaluating a biological sample obtained from the patient for the level of one or more of the above polypeptides, relative to a predetermined cut-off value. As used herein, suitable "biological samples" include blood, sera and urine.

The level of one or more of the above polypeptides may be evaluated using any binding agent specific for the polypeptide(s). A "binding agent," in the context of this invention, is any agent (such as a compound or a cell) that binds to a polypeptide as described above. As used herein, "binding" refers to a noncovalent association between two separate molecules (each of which may be free *(i.e.,* in solution) or present on the surface of a cell or a solid support), such that a "complex" is formed. Such a complex may be free or immobilized (either covalently or noncovalently) on a support material. The ability to bind may generally be evaluated by determining a binding constant for the formation of the complex. The binding constant is the value obtained when the concentration of the complex is divided by the product of the component concentrations. In general, two compounds are said to "bind" in the context of the present invention when the binding constant for complex formation exceeds about 10³ L/mol. The binding constant may be determined using methods well known to those of ordinary skill in the art.

Any agent that satisfies the above requirements may be a binding agent. For example, a binding agent may be a ribosome with or without a peptide component, an RNA molecule or a peptide. In a preferred embodiment, the binding partner is an antibody, or a fragment thereof. Such antibodies may be polyclonal, or monoclonal. In addition, the antibodies may be single chain, chimeric, CDR-grafted or humanized. Antibodies may be prepared by the methods described herein and by other methods well known to those of skill in the art.

There are a variety of assay formats known to those of ordinary skill in the art for using a binding partner to detect polypeptide markers in a sample. *See, e.g.,* Harlow and Lane, Antibodies: A Laboratory Manual, Cold Spring Harbor Laboratory, 1988. In a preferred embodiment, the assay involves the use of binding partner immobilized on a solid support to bind to and remove the polypeptide from the remainder of the sample. The bound polypeptide may then be detected using a second binding partner that contains a reporter group. Suitable second binding partners include antibodies that bind to the binding partner/polypeptide complex. Alternatively, a competitive assay may be utilized, in which a polypeptide is labeled with a reporter group and allowed to bind to the immobilized binding partner after incubation of the binding partner with the sample. The extent to which components of the sample inhibit the binding of the labeled polypeptide to the binding partner is indicative of the reactivity of the sample with the immobilized binding partner.

The solid support may be any material known to those of ordinary skill in the art to which the antigen may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the antigen and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (see, *e.g.,* Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a second antibody (containing a reporter group) capable of binding to a different site on the polypeptide is added. The amount of second antibody that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20^{™} (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i.e*., incubation time) is that period of time that is sufficient to detect the presence of polypeptide within a sample obtained from an individual with breast cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20^{™}. The second antibody, which contains a reporter group, may then be added to the solid support. Preferred reporter groups include enzymes (such as horseradish peroxidase), substrates, cofactors, inhibitors, dyes, radionuclides, luminescent groups, fluorescent groups and biotin. The conjugation of antibody to reporter group may be achieved using standard methods known to those of ordinary skill in the art.

The second antibody is then incubated with the immobilized antibody-polypeptide complex for an amount of time sufficient to detect the bound polypeptide. An appropriate amount of time may generally be determined by assaying the level of binding that occurs over a period of time. Unbound second antibody is then removed and bound second antibody is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of breast cancer, the signal detected from the reporter group that remains bound to the solid support is generally compared to a signal that corresponds to a predetermined cut-off value. In one preferred embodiment, the cut-off value is the average mean signal obtained when the immobilized antibody is incubated with samples from patients without breast cancer. In general, a sample generating a signal that is three standard deviations above the predetermined cut-off value is considered positive for breast cancer. In an alternate preferred embodiment, the cut-off value is determined using a Receiver Operator Curve, according to the method of Sackett et al., Clinical Epidemiology: A Basic Science for Clinical Medicine, Little Brown and Co., 1985, p. 106-7. Briefly, in this embodiment, the cut-off value may be determined from a plot of pairs of true positive rates *(i.e.,* sensitivity) and false positive rates (100%-specificity) that correspond to each possible cut-off value for the diagnostic test result. The cut-off value on the plot that is the closest to the upper left-hand corner (*i.e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for breast cancer.

In a related embodiment, the assay is performed in a flow-through or strip test format, wherein the antibody is immobilized on a membrane, such as nitrocellulose. In the flow-through test, polypeptides within the sample bind to the immobilized antibody as the sample passes through the membrane. A second, labeled antibody then binds to the antibody-polypeptide complex as a solution containing the second antibody flows through the membrane. The detection of bound second antibody may then be performed as described above. In the strip test format, one end of the membrane to which antibody is bound is immersed in a solution containing the sample. The sample migrates along the membrane through a region containing second antibody and to the area of immobilized antibody. Concentration of second antibody at the area of immobilized antibody indicates the presence of breast cancer. Typically, the concentration of second antibody at that site generates a pattern, such as a line, that can be read visually. The absence of such a pattern indicates a negative result. In general, the amount of antibody immobilized on the membrane is selected to generate a visually discernible pattern when the biological sample contains a level of polypeptide that would be sufficient to generate a positive signal in the two-antibody sandwich assay, in the format discussed above. Preferably, the amount of antibody immobilized on the membrane ranges from about 25 ng to about 1 µg, and more preferably from about 50 ng to about 500 ng. Such tests can typically be performed with a very small amount of biological sample.

Of course, numerous other assay protocols exist that are suitable for use with the antigens or antibodies of the present invention. The above descriptions are intended to be exemplary only.

In another embodiment, the above polypeptides may be used as markers for the progression of breast cancer. In this embodiment, assays as described above for the diagnosis of breast cancer may be performed over time, and the change in the level of reactive polypeptide(s) evaluated. For example, the assays may be performed every 24-72 hours for a period of 6 months to 1 year, and thereafter performed as needed. In general, breast cancer is progressing in those patients in whom the level of polypeptide detected by the binding agent increases over time. In contrast, breast cancer is not progressing when the level of reactive polypeptide either remains constant or decreases with time.

Antibodies for use in the above methods may be prepared by any of a variety of techniques known to those of ordinary skill in the art. *See, e.g.,* Harlow and Lane, Antibodies: A Laboratory Manual, Cold Spring Harbor Laboratory, 1988. In one such technique, an immunogen comprising the antigenic polypeptide is initially injected into any of a wide variety of mammals (e.g., mice, rats, rabbits, sheep and goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for the antigenic polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i.e*., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Monoclonal antibodies of the present invention may also be used as therapeutic reagents, to diminish or eliminate breast tumors. The antibodies may be used on their own (for instance, to inhibit metastases) or coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (e.g., covalently bonded) to a suitable monoclonal antibody either directly or indirectly (e.g., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (e.g., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e.g*., U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (e.g., U.S. Patent No. 4,489,710, to Spitler), by irradiation of a photolabile bond (e.g., U.S. Patent No. 4,625,014, to Senter et al.), by hydrolysis of derivatized amino acid side chains (e.g., U.S. Patent No. 4,638,045, to Kohn et al.), by serum complement-mediated hydrolysis (e.g., U.S. Patent No. 4,671,958, to Rodwell et al.), and acid-catalyzed hydrolysis (e.g., U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used.

A carrier may bear the agents in a variety of ways, including covalent bonding either directly or via a linker group. Suitable carriers include proteins such as albumins (e.g., U.S. Patent No. 4,507,234, to Kato et al.), peptides and polysaccharides such as aminodextran (e.g., U.S. Patent No. 4,699,784, to Shih et al.). A carrier may also bear an agent by noncovalent bonding or by encapsulation, such as within a liposome vesicle (e.g., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

Diagnostic reagents of the present invention may also comprise polynucleotide sequences encoding one or more of the above polypeptides, or one or more portions thereof. For example, at least two oligonucleotide primers may be employed in a polymerase chain reaction (PCR) based assay to amplify breast tumor-specific cDNA derived from a biological sample, wherein at least one of the oligonucleotide primers is specific for a DNA molecule encoding a breast tumor protein of the present invention. The presence of the amplified cDNA is then detected using techniques well known in the art, such as gel electrophoresis. Similarly, oligonucleotide probes specific for a DNA molecule encoding a breast tumor protein of the present invention may be used in a hybridization assay to detect the presence of an inventive polypeptide in a biological sample.

As used herein, the term "oligonucleotide primer/probe specific for a DNA molecule" means an oligonucleotide sequence that has at least about 60%, preferably at least about 75% and more preferably at least about 90%, identity to the DNA molecule in question. Oligonucleotide primers and/or probes which may be usefully employed in the inventive diagnostic methods preferably have at least about 10-40 nucleotides. In a preferred embodiment, the oligonucleotide primers comprise at least about 10 contiguous nucleotides of a DNA molecule having a partial sequence selected from SEQ ID NOS: 1- 94. Preferably, oligonucleotide probes for use in the inventive diagnostic methods comprise at least about 15 contiguous oligonucleotides of a DNA molecule having a partial sequence provided in SEQ ID NOS: 1- 94. Techniques for both PCR based assays and hybridization assays are well known in the art (see, for example, Mullis *et al. Ibid*; Ehrlich, *Ibid*). Primers or probes may thus be used to detect breast tumor-specific sequences in biological samples, including blood, urine and/or breast tumor tissue.
Aspects of the invention are defined in the numbered paragraphs below:
1. An isolated polypeptide comprising an immunogenic portion of a breast protein or a variant of said protein that differs only in conservative substitutions and/or modifications, wherein said protein comprises an amino acid sequence encoded by a polynucleotide molecule comprising a sequence selected from the group consisting of: (a) nucleotide sequences recited in SEQ ID NOS: 3, 10, 17, 24, 45-52, 55-67, 72, 73, and 89-94; (b) complements of said nucleotide sequences; and (c) sequences that hybridize to a sequence of (a) or (b) under moderately stringent conditions.
2. An isolated polynucleotide molecule comprising a nucleotide sequence encoding the polypeptide of paragraph 1.
3. An isolated polynucleotide molecule comprising a sequence provided in SEQ ID NOS: 3, 10, 17, 24, 45-52, 55-67, 72, 73, and 89-94.
4. An expression vector comprising a polynucleotide molecule according to any one of paragraphs 2 and 3.
5. A host cell transformed with the expression vector of paragraph 4.
6. The host cell of paragraph 5 wherein the host cell is selected from the group consisting of *E*. *coli,* yeast and mammalian cell lines.
7. A pharmaceutical composition comprising the polypeptide of paragraph 1 and a physiologically acceptable carrier.
8. A vaccine comprising the polypeptide of paragraph 1 and a non-specific immune response enhancer.
9. The vaccine of paragraph 8 wherein the non-specific immune response enhancer is an adjuvant.
10. A vaccine comprising a polynucleotide molecule of any one of paragraphs 2 and 3 and a non-specific immune response enhancer.
11. The vaccine of paragraph 10 wherein the non-specific immune response enhancer is an adjuvant.
12. A pharmaceutical composition for the treatment of breast cancer comprising a polypeptide and a physiologically acceptable carrier, the polypeptide comprising an immunogenic portion of a breast protein, wherein said protein comprises an amino acid sequence encoded by a polynucleotide molecule comprising a sequence selected from the group consisting of: (a) nucleotide sequences recited in SEQ ID NOS: 1, 2, 4-9, 11-16, 18-23, 25-44, 53, 54, 68-71, and 74-88; (b) complements of said nucleotide sequences; and (c) sequences that hybridize to a sequence of (a) or (b) under moderately stringent conditions.
13. A vaccine for the treatment of breast cancer comprising a polypeptide and a non-specific immune response enhancer, said polypeptide comprising an immunogenic portion of a breast protein, wherein said protein comprises an amino acid sequence encoded by a polynucleotide molecule comprising a sequence selected from the group consisting of: (a) nucleotide sequences recited in SEQ ID NOS: 1, 2, 4-9, 11-16, 18-23, 25-44, 53, 54, 68-71, and 74-88; (b) complements of said nucleotide sequences; and (c) sequences that hybridize to a sequence of (a) or (b) under moderately stringent conditions.
14. The vaccine of paragraph 13 wherein the non-specific immune response enhancer is an adjuvant.
15. A vaccine for the treatment of breast cancer comprising a polynucleotide molecule and a non-specific immune response enhancer, the polynucleotide molecule comprising a sequence selected from the group consisting of: (a) nucleotide sequences recited in SEQ ID NOS: 1, 2, 4-9, 11-16, 18-23, 25-44, 53, 54, 68-71, and 74-88; (b) complements of said nucleotide sequences; and (c) sequences that hybridize to a sequence of (a) or (b) under moderately stringent conditions.
16. The vaccine of paragraph 15, wherein the non-specific immune response enhancer is an adjuvant.
17. A pharmaceutical composition according to paragraphs 7 or 12, for use in the manufacture of a medicament for inhibiting the development of breast cancer in a patient.
18. A vaccine according to any one of paragraphs 8, 10, 13 or 15, for use in the manufacture of a medicament for inhibiting the development of breast cancer in a patient.
19. A fusion protein comprising at least one polypeptide according to paragraph 1.
20. A pharmaceutical composition comprising a fusion protein according to paragraph 19 and a physiologically acceptable carrier.
21. A vaccine comprising a fusion protein according to paragraph 19 and a non-specific immune response enhancer.
22. The vaccine of paragraph 21 wherein the non-specific immune response enhancer is an adjuvant.
23. A pharmaceutical composition according to paragraph 20, for use in manufacture of a medicament for inhibiting the development of breast cancer in a patient.
24. A vaccine according to paragraph 21, for use in the manufacture of a medicament for inhibiting the development of breast cancer in a patient.
25. A method for detecting breast cancer in a patient, comprising:
   (a) contacting a biological sample from a patient with a binding agent which is capable of binding to a polypeptide, the polypeptide comprising an immunogenic portion of a breast protein, wherein said protein comprises an amino acid sequence encoded by a polynucleotide molecule comprising a sequence selected from the group consisting of nucleotide sequences recited in SEQ ID NOS: 1-94, complements of said nucleotide sequences and sequences that hybridize to a sequence provided in SEQ ID NO: 1-94 under moderately stringent conditions; and
   (b) detecting in the sample a protein or polypeptide that binds to the binding agent, thereby detecting breast cancer in the patient.
26. The method of paragraph 25 wherein the binding agent is a monoclonal antibody.
27. The method of paragraph 26 wherein the binding agent is a polyclonal antibody.
28. A method for monitoring the progression of breast cancer in a patient, comprising:
   (a) contacting a biological sample from a patient with a binding agent that is capable of binding to a polypeptide, said polypeptide comprising an immunogenic portion of a breast protein, wherein said protein comprises an amino acid sequence encoded by a polynucleotide molecule comprising a sequence selected from the group consisting of nucleotide sequences recited in SEQ ID NOS: 1-94, complements of said nucleotide sequences and sequences that hybridize to a sequence provided in SEQ ID NO: 1-94 under moderately stringent conditions;
   (b) determining in the sample an amount of a protein or polypeptide that binds to the binding agent;
   (c) repeating steps (a) and (b); and
   (d) comparing the amount of polypeptide detected in steps (b) and (c) to monitor the progression of breast cancer in the patient.
29. A monoclonal antibody that binds to a polypeptide comprising an immunogenic portion of a breast protein or a variant of said protein that differs only in conservative substitutions and/or modifications, wherein said protein comprises an amino acid sequence encoded by a polynucleotide molecule comprising a sequence selected from the group consisting of: (a) nucleotide sequences recited in SEQ ID NOS: 3, 10, 17, 24, 45-52, 55-67, 72, 73, and 89-94: (b) complements of said nucleotide sequences; and (c) sequences that hybridize to a sequence of (a) or (b) under moderately stringent conditions.
30. A monoclonal antibody according to paragraph 29, for use in the manufacture of a medicament for inhibiting the development of breast cancer in a patient.
31. The monoclonal antibody of paragraph 30 wherein the monoclonal antibody is conjugated to a therapeutic agent.
32. A method for detecting breast cancer in a patient comprising:
   (a) contacting a biological sample from a patient with at least two oligonucleotide primers in a polymerase chain reaction, wherein at least one of the oligonucleotides is specific for a polynucleotide molecule encoding a polypeptide comprising an immunogenic portion of a breast protein, said protein comprising an amino acid sequence encoded by a polynucleotide molecule comprising a sequence selected from the group consisting of nucleotide sequences recited in SEQ ID NO: 1-94, complements of said nucleotide sequences and sequences that hybridize to a sequence of SEQ ID NO: 1-94 under moderately stringent conditions; and
   (b) detecting in the sample a polynucleotide sequence that amplifies in the presence of the oligonucleotide primers, thereby detecting breast cancer.
33. The method of paragraph 32, wherein at least one of the oligonucleotide primers comprises at least about 10 contiguous nucleotides of a polynucleotide molecule comprising a sequence selected from SEQ ID NOS: 1-94.
34. A diagnostic kit comprising:
   (a) one or more monoclonal antibodies of paragraph 29; and
   (b) a detection reagent.
35. A diagnostic kit comprising:
   (a) one or more monoclonal antibodies that bind to a polypeptide encoded by a polynucleotide molecule comprising a nucleotide sequence selected from the group consisting of SEQ ID NOS: 1, 2, 4-9, 11-16, 18-23, 25-44, 53, 54, 68-71, and 74-88, complements of said sequences and sequences that hybridize to a sequence of SEQ ID NO: 1, 2, 4-9, 11-16, 18-23, 25-44, 53, 54, 68-71, or 74-88 under moderately stringent conditions; and
   (b) a detection reagent.
36. The kit of paragraphs 34 or 35 wherein the monoclonal antibodies are immobilized on a solid support.
37. The kit of paragraph 36 wherein the solid support comprises nitrocellulose, latex or a plastic material.
38. The kit of paragraphs 34 or 35 wherein the detection reagent comprises a reporter group conjugated to a binding agent.
39. The kit of paragraph 38 wherein the binding agent is selected from the group consisting of anti-immunoglobulins, Protein G, Protein A and lectins.
40. The kit of paragraph 38 wherein the reporter group is selected from the group consisting of radioisotopes, fluorescent groups, luminescent groups, enzymes, biotin and dye particles.
41. A diagnostic kit comprising at least two oligonucleotide primers, at least one of the oligonucleotide primers being specific for a polynucleotide molecule encoding a polypeptide comprising an immunogenic portion of a breast protein, said protein comprising an amino acid sequence encoded by a polynucleotide molecule comprising a sequence selected from the group consisting of nucleotide sequences recited in SEQ ID NOS: 1-94, complements of said nucleotide sequences and sequences that hybridize to a sequence of SEQ ID NO: 1-94 under moderately stringent conditions.
42. A diagnostic kit of paragraph 41 wherein at least one of the oligonucleotide primers comprises at least about 10 contiguous nucleotides of a polynucleotide molecule comprising a sequence selected from SEQ ID NOS: 1-94.
43. A method for detecting breast cancer in a patient, comprising:
   (a) obtaining a biological sample from the patient;
   (b) contacting the biological sample with an oligonucleotide probe specific for a polynucleotide molecule encoding a polypeptide comprising an immunogenic portion of a breast protein, said protein comprising an amino acid sequence encoded by a polynucleotide molecule comprising a sequence selected from the group consisting of nucleotide sequences recited in SEQ ID NOS: 1-94, complements of said nucleotide sequences and sequences that hybridize to a sequence of SEQ ID NO: 1-94 under moderately stringent conditions; and
   (c) detecting in the sample a polynucleotide sequence that hybridizes to the oligonucleotide probe, thereby detecting breast cancer in the patient.
44. The method of paragraph 43 wherein the oligonucleotide probe comprises at least about 15 contiguous nucleotides of a polynucleotide molecule comprising a sequence selected from the group consisting of SEQ ID NOS: 1-94.
45. A diagnostic kit comprising an oligonucleotide probe specific for a polynucleotide molecule encoding a polypeptide comprising an immunogenic portion of a breast protein, said protein comprising an amino acid sequence encoded by a polynucleotide molecule comprising a sequence selected from the group consisting of nucleotide sequences recited in SEQ ID NOS: 1-94, complements of said nucleotide sequences, and sequences that hybridize to a sequence of SEQ ID NO: 1-94 under moderately stringent conditions.
46. The diagnostic kit of paragraph 45, wherein the oligonucleotide probe comprises at least about 15 contiguous nucleotides of a polynucleotide molecule comprising a sequence selected from the group consisting of SEQ ID NOS: 1-94.
47. Peripheral blood cells from a patient incubated in the presence of at least one polypeptide of paragraph 1, such that T cells proliferate, for use in the manufacture of a medicament for treating breast cancer in a patient.
48. The blood cells of paragraph 47 wherein the T cells is repeated one or more times.
49. A composition for the treatment of breast cancer in a patient, comprising T cells proliferated in the presence of a polypeptide of paragraph 1, in combination with a pharmaceutically acceptable carrier.
50. An antigen presenting cells incubated in the presence of at least one polypeptide of paragraph 1, for use in the manufacture of a medicament for treating breast cancer in a patient.
51. The cells of paragraph 50 wherein the antigen presenting cells are selected from the group consisting of dendritic and macrophage cells.
52. A composition for the treatment of breast cancer in a patient, comprising antigen presenting cells incubated in the presence of a polypeptide of paragraph 1, in combination with a pharmaceutically acceptable carrier.

The following Examples are offered by way of illustration and not by way of limitation.

### EXAMPLES

### Example 1

### ISOLATION AND CHARACTERIZATION OF BREAST TUMOR POLYPEPTIDES

This Example describes the isolation of breast tumor polypeptides from a breast tumor cDNA library.

A human breast tumor cDNA expression library was constructed from a pool of breast tumor poly A⁺ RNA from three patients using a Superscript Plasmid System for cDNA Synthesis and Plasmid Cloning kit (BRL Life Technologies, Gaithersburg, MD 20897) following the manufacturer's protocol. Specifically, breast tumor tissues were homogenized with polytron (Kinematica, Switzerland) and total RNA was extracted using Trizol reagent (BRL Life Technologies) as directed by the manufacturer. The poly A⁺ RNA was then purified using a Qiagen oligotex spin column mRNA purification kit (Qiagen, Santa Clarita, CA 91355) according to the manufacturer's protocol. First-strand cDNA was synthesized using the NotI/Oligo-dT18 primer. Double-stranded cDNA was synthesized, ligated with EcoRI/BstX I adaptors (Invitrogen, Carlsbad, CA) and digested with NotI. Following size fractionation with Chroma Spin-1000 columns (Clontech, Palo Alto, CA 94303), the cDNA was ligated into the EcoRI/NotI site of pCDNA3.1 (Invitrogen, Carlsbad, CA) and transformed into ElectroMax *E. coli* DH10B cells (BRL Life Technologies) by electroporation.

Using the same procedure, a normal human breast cDNA expression library was prepared from a pool of four normal breast tissue specimens. The cDNA libraries were characterized by determining the number of independent colonies, the percentage of clones that carried insert, the average insert size and by sequence analysis. The breast tumor library contained 1.14 x 10⁷ independent colonies, with more than 90% of clones having a visible insert and the average insert size being 936 base pairs. The normal breast cDNA library contained 6 x 10⁶ independent colonies, with 83% of clones having inserts and the average insert size being 1015 base pairs. Sequencing analysis showed both libraries to contain good complex cDNA clones that were synthesized from mRNA, with minimal rRNA and mitochondrial DNA contamination sequencing.

cDNA library subtraction was performed using the above breast tumor and normal breast cDNA libraries, as described by Hara et al. (Blood, 84:189-199, 1994) with some modifications. Specifically, a breast tumor-specific subtracted cDNA library was generated as follows. Normal breast cDNA library (70 µg) was digested with EcoRI, NotI, and SfuI, followed by a filling-in reaction with DNA polymerase Klenow fragment. After phenol-chloroform extraction and ethanol precipitation, the DNA was dissolved in 100 µl of H₂O, heat-denatured and mixed with 100 µl (100 µg) of Photoprobe biotin (Vector Laboratories, Burlingame, CA), the resulting mixture was irradiated with a 270 W sunlamp on ice for 20 minutes. Additional Photoprobe biotin (50 µl) was added and the biotinylation reaction was repeated. After extraction with butanol five times, the DNA was ethanol-precipitated and dissolved in 23 µl H₂O to form the driver DNA.

To form the tracer DNA, 10 µg breast tumor cDNA library was digested with BamHI and XhoI, phenol chloroform extracted and passed through Chroma spin-400 columns (Clontech). Following ethanol precipitation, the tracer DNA was dissolved in 5 µl H₂O. Tracer DNA was mixed with 15 µl driver DNA and 20 µl of 2 x hybridization buffer (1.5 M NaCl/10 mM EDTA/50 mM HEPES pH 7.5/0.2% sodium dodecyl sulfate), overlaid with mineral oil, and heat-denatured completely. The sample was immediately transferred into a 68 °C water bath and incubated for 20 hours (long hybridization [LH]). The reaction mixture was then subjected to a streptavidin treatment followed by phenol/chloroform extraction. This process was repeated three more times. Subtracted DNA was precipitated, dissolved in 12 µl H₂O, mixed with 8 µl driver DNA and 20 µl of 2 x hybridization buffer, and subjected to a hybridization at 68 °C for 2 hours (short hybridization [SH]). After removal of biotinylated double-stranded DNA, subtracted cDNA was ligated into BamHI/XhoI site of chloramphenicol resistant pBCSK⁺ (Stratagene, La Jolla, CA 92037) and transformed into ElectroMax *E. coli* DH10B cells by electroporation to generate a breast tumor specific subtracted cDNA library.

To analyze the subtracted cDNA library, plasmid DNA was prepared from 100 independent clones, randomly picked from the subtracted breast tumor specific library and characterized by DNA sequencing with a Perkin Elmer/Applied Biosystems Division Automated Sequencer Model 373A (Foster City, CA). Thirty-eight distinct cDNA clones were found in the subtracted breast tumor-specific cDNA library. The determined 3' cDNA sequences for 14 of these clones are provided in SEQ ID NO: 1-14, with the corresponding 5' cDNA sequences being provided in SEQ ID NO: 15-28, respectively. The determined one strand (5' or 3') cDNA sequences for the remaining clones are provided in SEQ ID NO: 29-52. Comparison of these cDNA sequences with known sequences in the gene bank using the EMBL and GenBank databases (Release 97) revealed no significant homologies to the sequences provided in SEQ ID NO: 3, 10, 17, 24 and 45-52. The sequences provided in SEQ ID NO: 1, 2, 4-9, 11-16, 18-23, 25-41 , 43 and 44 were found to show at least some degree of homology to known human genes. The sequence of SEQ ID NO: 42 was found to show some homology to a known yeast gene.

Data was analyzed using Synteni provided GEMTOOLS Software. Twenty one distinct cDNA clones were found to be over-expressed in breast tumor and expressed at low levels in all normal tissues tested. The determined partial cDNA sequences for these clones are provided in SEQ ID NO: 53- 73. Comparison of the sequences of SEQ ID NO: 53, 54, and 68-71 with those in the gene bank as described above, revealed some homology to previously identified human genes. No significant homologies were found to the sequences of SEQ ID NO: 55-67, 72 (referred to as JJ 9434,7117), and 73 (referred to as B535S).

In a further experiment, cDNA fragments analyzed by DNA microarray were obtained from two subtraction libraries derived by conventional subtraction, as described above. In one instance the tester was derived from primary breast tumors. In the second instance, a metastatic breast tumor was employed as the tester. Drivers consisted of normal breast.

cDNA fragments from these two libraries were submitted as templates for DNA microarray analysis. DNA chips were analyzed by hybridizing with fluorescent probes derived from mRNA from both tumor and normal tissues. Analysis of the data was accomplished by creating three groups from the sets of probes. The composition of these probe groups, referred to as Breast Tumor/mets, Normal non-breast tissues, and Metastatic breast tumors. Two comparisons were performed using the modified Gemtools analysis. The first comparison was to identify templates with elevated expression in breast tumors. The second was to identify templates not recovered in the first comparison that yielded elevated expression in metastatic breast tumors. An arbitrary level of increased expression (mean of tumor expression versus the mean of normal tissue expression) was set at approximately 2.2.

In the first round of comparison to identify overexpression in breast tumors, two novel gene sequences were identified, hereinafter referred to as B534S and B538S (SEQ ID NO: 89 and 90), and six sequences that showed some degree of homology to previously identified genes (SEQ ID NO: 74-79). Additionally, in a second comparison to identify elevated expression in metastatic breast tumors, five novel sequences were identified, hereinafter referred to as B535S (overexpressed in this analysis as well as what was described above), B542S, B543S, P501S, and B541S (SEQ ID NO: 73, and 91-94), as well as nine gene sequences that showed some homology to known genes (SEQ ID NO: 80-88). Clone B534S and B538S (SEQ ID NO: 89 and 90) were shown to be overexpressed in both breast tumors and metastatic breast tumors.

### Example 2

### GENERATION OF HUMAN CD8+ CYTOTOXIC T-CELLS THAT RECOGNIZE ANTIGEN PRESENTING CELLS EXPRESSING BREAST TUMOR ANTIGENS

This Example illustrates the generation of T cells that recognize target cells expressing the antigen B511S, also known as 1016-F8 (SEQ ID NO: 56). Human CD8+ T cells were primed *in-vitro* to the B511S gene product using dendritic cells infected with a recombinant vaccinia virus engineered to express B511S as follows (also see Yee et al., Journal of Immunology (1996) 157 (9):4079-86). Dendritic cells (DC) were generated from peripheral blood derived monocytes by differentiation for 5 days in the presence of 50 µg/ml GMCSF and 30 µg/ml IL-4. DC were harvested, plated in wells of a 24-well plate at a density of 2 x 10⁵ cells/well and infected for 12 hours with B511S expressing vaccinia at a multiplicity of infection of 5. DC were then matured overnight by the addition of 3 µg/ml CD40-Ligand and UV irradiated at 100µW for 10 minutes. CD8+ T cells were isolated using magnetic beads, and priming cultures were initiated in individual wells (typically in 24 wells of a 24-well plate) using 7 x 10⁵ CD8+ T cells and 1 x 10⁶ irradiated CD8-depleted PBMC; IL-7 at 10 ng/ml was added to cultures at day 1. Cultures were re-stimulated every 7-10 days using autologous primary fibroblasts retrovirally transduced with B511 S and the costimulatory molecule B7.1. Cultures were supplemented at day 1 with 15 I.U. of IL-2. Following 4 such stimulation cycles, CD8+ cultures were tested for their ability to specifically recognize autologous fibroblasts transduced with B511 S using an interferon-γ Elispot assay (see Lalvani et al J. Experimental Medicine (1997) 186:859-965). Briefly, T cells from individual microcultures were added to 96-well Elispot plates that contained autologous fibroblasts transduced to express either B511S or as a negative control antigen EGFP, and incubated overnight at 37° C; wells also contained IL-12 at 10 ng/ml. Cultures were identified that specifically produced interferon-γ only in response to B511S transduced fibroblasts; such lines were further expanded and also cloned by limiting dilution on autologous B-LCL retrovirally transduced with B511S. Lines and clones were identified that could specifically recognize autologous B-LCL transduced with B511 S but not autologous B-LCL transduced with the control antigens EGFP or HLA-A3. An example demonstrating the ability of human CTL cell lines derived from such experiments to specifically recognize and lyse B511S expressing targets is presented in Figure 1.

### Example 3

### SYNTHESIS OF POLYPEPTIDES

Polypeptides may be synthesized on an Perkin Elmer/Applied Biosystems Division 430A peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugation, binding to an immobilized surface, or labeling of the peptide. Cleavage of the peptides from the solid support may be carried out using the following cleavage mixture: trifluoroacetic acid:ethanedithiol:thioanisole:water:phenol (40:1:2:2:3). After cleaving for 2 hours, the peptides may be precipitated in cold methyl-t-butyl-ether. The peptide pellets may then be dissolved in water containing 0.1% trifluoroacetic acid (TFA) and lyophilized prior to purification by C18 reverse phase HPLC. A gradient of 0%-60% acetonitrile (containing 0.1% TFA) in water (containing 0.1% TFA) may be used to elute the peptides. Following lyophilization of the pure fractions, the peptides may be characterized using electrospray or other types of mass spectrometry and by amino acid analysis.

From the foregoing, it will be appreciated that, although specific embodiments of the invention have been described herein for the purposes of illustration, various modifications may be made without deviating from the spirit and scope of the invention.

## Claims

1. An isolated polypeptide comprising an immunogenic portion of a breast protein or a variant of said protein that differs only in conservative substitutions and/or modifications, wherein said protein comprises an amino acid sequence encoded by a polynucleotide molecule comprising a sequence selected from the group consisting of: (a) nucleotide sequence recited in SEQ ID NO: 55; (b) a complement of said nucleotide sequence; and (c) sequences that hybridize to a sequence of (a) or (b) under moderately stringent conditions.

2. An isolated polynucleotide molecule comprising a nucleotide sequence encoding the polypeptide of claim 1.

3. An isolated polynucleotide molecule comprising a sequence provided in SEQ ID NO: 55.

4. An expression vector comprising a polynucleotide molecule according to any one of claims 2 and 3.

5. A host cell transformed with the expression vector of claim 4.

6. The host cell of claim 5 wherein the host cell is selected from the group consisting of *E*. *coli,* yeast and mammalian cell lines.

7. A fusion protein comprising at least one polypeptide according to claim 1.

8. A pharmaceutical composition comprising the polypeptide of claim 1, or a fusion protein according to claim 7, and a physiologically acceptable carrier.

9. A vaccine comprising the polypeptide of claim 1, or a fusion protein according to claim 7, and a non-specific immune response enhancer.

10. A vaccine comprising a polynucleotide molecule of any one of claims 2 and 3 and a non-specific immune response enhancer.

11. The vaccine of claim 9 or 10 wherein the non-specific immune response enhancer is an adjuvant.

12. A pharmaceutical composition according to claim 8, for use in inhibiting the development of breast cancer in a patient.

13. A vaccine according to claim 9 or 10, for use in inhibiting the development of breast cancer in a patient.

14. A method for detecting breast cancer in a patient, comprising:
(a) contacting a biological sample from a patient with a binding agent which is capable of binding to a polypeptide, the polypeptide comprising an immunogenic portion of a breast protein, wherein said protein comprises an amino acid sequence encoded by a polynucleotide molecule comprising a sequence selected from the group consisting of nucleotide sequence recited in SEQ ID NO: 55, a complement of said nucleotide sequence and sequences that hybridize to a sequence provided in SEQ ID NO: 55 under moderately stringent conditions; and
(b) detecting in the sample a protein or polypeptide that binds to the binding agent, thereby detecting breast cancer in the patient.

15. The method of claim 14 wherein the binding agent is a monoclonal antibody.

16. The method of claim 14 wherein the binding agent is a polyclonal antibody.

17. A method for monitoring the progression of breast cancer in a patient, comprising:
(a) contacting a biological sample from a patient with a binding agent that is capable of binding to a polypeptide, said polypeptide comprising an immunogenic portion of a breast protein, wherein said protein comprises an amino acid sequence encoded by a polynucleotide molecule comprising a sequence selected from the group consisting of nucleotide sequence recited in SEQ ID NO: 55, a complement of said nucleotide sequence and sequences that hybridize to a sequence provided in SEQ ID NO: 55 under moderately stringent conditions;
(b) determining in the sample an amount of a protein or polypeptide that binds to the binding agent;
(c) repeating steps (a) and (b); and
(d) comparing the amount of polypeptide detected in steps (b) and (c) to monitor the progression of breast cancer in the patient.

18. A monoclonal antibody that binds to a polypeptide comprising an immunogenic portion of a breast protein or a variant of said protein that differs only in conservative substitutions and/or modifications, wherein said protein comprises an amino acid sequence encoded by a polynucleotide molecule comprising a sequence selected from the group consisting of: (a) nucleotide sequence recited in SEQ ID NO: 55: (b) a complement of said nucleotide sequence; and (c) sequences that hybridize to a sequence of (a) or (b) under moderately stringent conditions.

19. A monoclonal antibody according to claim 18, for use in inhibiting the development of breast cancer in a patient.

20. The monoclonal antibody of claim 19 wherein the monoclonal antibody is conjugated to a therapeutic agent.

21. A method for detecting breast cancer in a patient comprising:
(a) contacting a biological sample from a patient with an oligonucleotide probe specific for a polynucleotide molecule encoding a polypeptide comprising an immunogenic portion of a breast protein, or with at least two oligonucleotide primers in a polymerase chain reaction, wherein at least one of the oligonucleotides is specific for a polynucleotide molecule encoding a polypeptide comprising an immunogenic portion of a breast protein, said protein comprising an amino acid sequence encoded by a polynucleotide molecule comprising a sequence selected from the group consisting of nucleotide sequence recited in SEQ ID NO: 55, a complement of said nucleotide sequence and sequences that hybridize to a sequence of SEQ ID NO: 55 under moderately stringent conditions; and
(b) detecting in the sample a polynucleotide sequence that hybridizes to the oligonucleotide probe, or that amplifies in the presence of the oligonucleotide primers, thereby detecting breast cancer.

22. The method of claim 21, wherein the oligonucleotide probe comprises at least about 15 contiguous nucleotides, or at least one of the oligonucleotide primers comprises at least about 10 contiguous nucleotides, of a polynucleotide molecule comprising a sequence of SEQ ID NO: 55.

23. A diagnostic kit comprising:
(a) one or more monoclonal antibodies of claim 18; and
(b) a detection reagent.

24. The kit of claim 23 wherein the monoclonal antibodies are immobilized on a solid support.

25. The kit of claim 24 wherein the solid support comprises nitrocellulose, latex or a plastic material.

26. The kit of claim 23 wherein the detection reagent comprises a reporter group conjugated to a binding agent.

27. The kit of claim 26 wherein the binding agent is selected from the group consisting of anti-immunoglobulins, Protein G, Protein A and lectins.

28. The kit of claim 26 wherein the reporter group is selected from the group consisting of radioisotopes, fluorescent groups, luminescent groups, enzymes, biotin and dye particles.

29. A diagnostic kit comprising an oligonucleotide probe specific for a polynucleotide molecule encoding a polypeptide comprising an immunogenic portion of a breast protein, or at least two oligonucleotide primers, at least one of the oligonucleotide primers being specific for a polynucleotide molecule encoding a polypeptide comprising an immunogenic portion of a breast protein, said protein comprising an amino acid sequence encoded by a polynucleotide molecule comprising a sequence selected from the group consisting of nucleotide sequence recited in SEQ ID NO: 55, a complement of said nucleotide sequence and sequences that hybridize to a sequence of SEQ ID NO: 55 under moderately stringent conditions.

30. A diagnostic kit of claim 29 wherein the oligonucleotide probe comprises at least about 15 contiguous nucleotides, or at least one of the oligonucleotide primers comprises at least about 10 contiguous nucleotides, of a polynucleotide molecule comprising a sequence of SEQ ID NO: 55.

31. Peripheral blood cells from a patient incubated in the presence of at least one polypeptide of claim 1, such that T cells proliferate, for use in treating breast cancer in a patient.

32. The blood cells of claim 31 wherein the T cells is repeated one or more times.

33. A composition for the treatment of breast cancer in a patient, comprising T cells proliferated in the presence of a polypeptide of claim 1, in combination with a pharmaceutically acceptable carrier.

34. An antigen presenting cells incubated in the presence of at least one polypeptide of claim 1, for use in treating breast cancer in a patient.

35. The cells of claim 34 wherein the antigen presenting cells are selected from the group consisting of dendritic and macrophage cells.

36. A composition for the treatment of breast cancer in a patient, comprising antigen presenting cells incubated in the presence of a polypeptide of claim 1, in combination with a pharmaceutically acceptable carrier.


SEQUENCE LISTING

<110> Corixa Corporation

<120> COMPOUNDS FOR IMMUNOTHERAPY AND
DIAGNOSIS OF BREAST CANCER AND METHODS FOR THEIR USE

<130> P/50121.EP02/NT

<140> Divisional of EP 98964884.5
<141> 22 December 1998

<150> EP 98964884.5
<151> 22 December 1998

<150> PCT/US98/27416
<151> 22 December 1998

<150> US 08/998,253
<151> 24 December 1997

<150> US 08/998,255
<151> 24 December 1997

<150> US 09/118,627
<151> 17 July 1998

<150> US 09/118,554
<151> 17 July 1998

<160> 94

<170> FastSEQ for Windows Version 3.0

<210> 1
<211> 402
<212> DNA
<213> Homo sapien

<220>
<221> misc_feature
<222> (1)...(402)
<223> n = A,T,C or G

<400> 1
tttttttttt tttttaggag aactgaatca aacagatttt attcaacttt ttagatgagg 60
aaaacaaatn atacgaaatn ngtcataaga aatgctttct tataccacta tctcaaacca 120
ctttcaatat tttacaaaat gctcacgcag caaatatgaa aagctncaac acttcccttt 180
gttaacttgc tgcaatnaat gcaactttaa canacataca aatttcttct gtatcttaaa 240
agttnaatta ctaattttaa tgatnttnct caagatnttt attcatatac ttttaatgac 300
tcnttgccna tacatacnta ttttctttac ttttttttta cnatnggcca acagctttca 360
ngcagnccnc aaaaatctta ccggttaatt acacggggtt gt 402

<210> 2
<211> 424
<212> DNA
<213> Homo sapien

<220>
<221> misc_feature
<222> (1)...(424)
<223> n = A,T,C or G

<400> 2
tttttttttt ttttttaaag gtacacattt ctttttcatt ctgtttnatg cagcaaataa 60
ttcgttggca tcttctctgt gatgggcagc ttgctaaaat tanactcagg ccccttagct 120
ncatttccaa ctnagcccac gctttcaacc nngccnaaca aagaaaatca gttngggtta 180
aattctttgc tgganacaaa gaactacatt cctttgtaaa tnatgctttg tttgctctgt 240

gcaaacncag attgaaggga anaagganac ttntggggac ggaaacaact ngnagaagca 300
gganccgccc agggncattt cctcaccatg cttaatcttg cnctcacttg cngggcacca 360
ttaaacttgg tgcaaaaggc gcaattggtg nanggaaccc cacaccttcc ttaaaaagca 420
gggc 424

<210> 3
<211> 421
<212> DNA
<213> Homo sapien

<220>
<221> misc_feature
<222> (1)...(421)
<223> n = A,T,C or G

<400> 3
tttttttttt tttttcccaa tttaaaaaag cctttttcat acttcaatta caccanactt 60
aatnatttca tgagtaaatc ngacattatt atttnaaaat ttgcatattt aaaatttgna 120
tcanttactt ccagactgtt tgcanaatga agggaggatc actcaagngc tgatctcnca 180
ctntctgcag tctnctgtcc tgtgcccggn ctaatggatc gacactanat ggacagntcn 240
cagatcttcc gttcttntcc cttccccaat ttcncaccnc tccccttctt ncccggatcn 300
tttggggaca tgntaatttt gcnatcctta aaccctgccc gccangggtc ccnanctcag 360
gggtggttaa tgttcgncng gcttnttgac cncctgcgcc ctttnantcc naaccccaag 420
c 421

<210> 4
<211> 423
<212> DNA
<213> Homo sapien

<220>
<221> misc_feature
<222> (1)...(423)
<223> n = A,T,C or G

<400> 4
tttttttatt tttttttcta tttntnntat ttnntgnggt tcctgtgtgt aattagnang 60
tgtgtatgcg tangtacnta tgtntgcata tttaacctgt tncctttcca tttttaaaat 120
aaaatctcaa natngtantt ggttnatggg agtaaanaga gactatngat naattttaac 180
atggacacng tgaaatgtag ccgctnatca ntttaaaact tcattttgaa ggccttttnc 240
cctccnaata aaaatnccng gccctactgg gttaagcaac attgcatntc taaagaaacc 300
acatgcanac nagttaaacc tgtgnactgg tcangcaaac cnanntggaa nanaagggnn 360
ttcnccccan ggacantcng aattttttta acaaattacn atnccccccc ngggggagcc 420
tgt 423

<210> 5
<211> 355
<212> DNA
<213> Homo sapien

<220>
<221> misc_feature
<222> (1)...(355)
<223> n = A,T,C or G

<400> 5
acgaccacct natttcgtat ctttcaactc ttttcgaccg gacctcttat tcggaagcgt 60
tccaggaaga caggtctcaa cttagggatc agatcacgtt atcaacgctc tgggatcgct 120
gcaacctggc acttcaagga agtgcaccga tnacgtctag accggccaac acagatctag 180
aggtggccaa ctgatcactg taggagctga ctggcaanan tcaaccgggc cccaaccnag 240
agtgaccaan acnaccattn aggatcaccc acaggcactc ctcgtcctag ggccaaccna 300
ccaaacggct ggccaatggg ggggtttaat atttggttna aaaattgatt ttaaa 355

<210> 6
<211> 423
<212> DNA
<213> Homo sapien

<220>
<221> misc_feature
<222> (1)...(423)
<223> n = A,T,C or G

<400> 6
tttttttttt tttttggaca ggaagtaaaa tttattggtn antattaana ggggggcagc 60
acattggaag ccctcatgan tgcagggccc gccacttgtc cagagggcca cnattgggga 120
tgtacttaac cccacagccn tctgggatna gccgcttttc agccaccatn tcttcaaatt 180
catcagcatt aaacttggta aanccccact tctttaagat ntgnatcttc tggcggccag 240
naaacttgaa cttggccctg cgcagggcct caatcacatg ctccttgttc tgcagcttgg 300
tgcgnaagga cntaatnact tggccnatgt gaaccctggc cacantgccc tggggctttc 360
caaaggcacc tcgcaagcct ntttggancc tgnccgcccc ngcacaggga caacatcttg 420
ttt 423

<210> 7
<211> 410
<212> DNA
<213> Homo sapien

<220>
<221> misc_feature
<222> (1)...(410)
<223> n = A,T,C or G

<400> 7
ttcgcactgg ctaaaacaaa ccgccttgca aagttngaaa aatttatcaa tggaccaaat 60
aatgctcata tccnacaagt tggtgaccgt tnttatnata aaaaaatgta tnatgctcct 120
nanttgttgt acaataatgt tccaatttng gacnttcggc atctaccctg gttcacctgg 180
gtaaatatca ggcagctttt gatggggcta ggaaagctaa cagtactcga acatgggaaa 240
gaggtctgct tcgccngtgt anatgggaaa naattccgtc ttgctcngat ttgtggactt 300
catattgttg tacatgcaga tgaatnngaa gaacttgtca actactatca ggatcgtggc 360
tttttnnaaa agctnatcac catgttggaa gcggcactng gacttgagcg 410

<210> 8
<211> 274
<212> DNA
<213> Homo sapien

<220>
<221> misc_feature
<222> (1)...(274)
<223> n = A,T,C or G

<400> 8
tttttttttt tttttaggtc atacatattt tttattataa canatatntg tatatacata 60
taatatatgt gtatatatcc acgtgtgtgt gtgtgtatca aaaacaacan aantttagtg 120
atctatatct ntngctcaca tatgcatggg agataccagt aaaaaataag tnaatctcca 180
taatatgttt taaaactcan anaaatcnga gagactnaaa gaaaacgttn atcannatga 240
ttgtngataa tcttgaanaa tnacnaaaac atat 274

<210> 9
<211> 322
<212> DNA
<213> Homo sapien

<220>
<221> misc_feature
<222> (1)...(322)
<223> n = A,T,C or G

<400> 9
tttttttttt ttttgtgcct tattgcaccg gcnanaactt ctagcactat attaaactca 60
ataagagtga taagtgtgaa aatccttgcc ttctctttaa tcttaatgna naggcatctg 120
gtttttcacc attaantgta ataatggctn tatgtatttt tatnnatggt cttnatggag 180
ttaaaaaagt tttcctctnt ccctngttat ctaanagttt tnatcaaaaa tgggtataat 240
atttngttca gtacttttnc ctgcacctat agatatgatn ctgttatttt ttcttcttng 300
cctnnanata tgatggatna ca 322

<210> 10
<211> 425
<212> DNA
<213> Homo sapien

<220>
<221> misc_feature
<222> (1)...(425)
<223> n = A,T,C or G

<400> 10
tttttttttt tttttattct gcagccatta aatgctgaac actagatnct tatttgtgga 60
ggtcacaaaa taagtacaga atatnacaca cgccctgccc ataaaaagca cagctcccag 120
ttctatattt acaatatctc tggaattcca ccttcccttc taatttgact aatatttctg 180
cttctcaggc agcagcgcct tctggcaacc ataagaacca acntgnggac taggtcggtg 240
ggccaaggat caggaaacag aanaatggaa gnagcccccn tgacnctatt aanctntnaa 300
actatctnaa ctgctagttt tcaggcttta aatcatgtaa natacgtgtc cttnttgctg 360
caaccggaag catcctagat ggtacactct ctccaggtgc caggaaaaga tcccaaatng 420
caggn 425

<210> 11
<211> 424
<212> DNA
<213> Homo sapien

<220>
<221> misc_feature
<222> (1)...(424)
<223> n = A,T,C or G

<400> 11
ttttnttant ttttttancc nctnntccnn tntgttgnag ggggtaccaa atttctttat 60
ttaaaggaat ggtacaaatc aaaaaactta atttaatttt tnggtacaac ttatagaaaa 120
ggttaaggaa accccaacat gcatgcactg ccttggtaac cagggnattc ccccncggct 180
ntggggaaat tagcccaang ctnagctttc attatcactn tcccccaggg tntgcttttc 240
aaaaaaattt nccgccnagc cnaatccggg cnctcccatc tggcgcaant tggtcacttg 300
gtcccccnat tctttaangg cttncacctn ctcattcggg tnatgtgtct caattaaatc 360
ccacngatgg gggtcatttt tntcnnttag ccagtttgtg nagttccgtt attganaaaa 420
ccan 424

<210> 12
<211> 426
<212> DNA
<213> Homo sapien

<220>
<221> misc_feature
<222> (1)...(426)
<223> n = A,T,C or G

<400> 12
tttttttttt ttttncttaa aagcttttat ctcctgctta cattacccat ctgttcttgc 60
atgttgtctg ctttttccac tagagccctt aacaacttaa tcatggttat tttaagggct 120
ctaataattc cnaaactggt atcataaata agtctcgttc tnatgcttgt tttctctcta 180
tcacactgtg ttngttgctt tttnacatgc tttgtaattt ttggctgaaa gctgaaaaat 240
nacatacctg gttntacaac ctgaggtaan cagccttnta gtgtgaggtt ttatatntta 300
ctggctaaga gctnggcnct gttnantant tgttgtanct ntatatgcca naggctttna 360
tttccnctng tgtccttgct tnagtacccc attnttttag ggttccccta naaactctat 420
ctnaat 426

<210> 13
<211> 419
<212> DNA
<213> Homo sapien

<220>
<221> misc_feature

<222> (1)...(419)
<223> n = A,T,C or G

<400> 13
tttttttttt tttttnagat agactctcac tctttcgccc aggctggagt gcagtggcgc 60
aatcaaggct cactgcaacc tctgccttat aaagcatttn ctaaaggtac aagctaaatt 120
ttaaaaatat ctctncacaa ctaatgtata acaaaaatta gttctacctc ataaacncnt 180
ggctcagccc tcgnaacaca tttccctgtt ctcaactgat gaacactcca naaacagaac 240
anatntaagc ttttccaggc ccagaaaagc tcgcgagggg atttgctntg tgtgtgacac 300
acttgccacc ctgtggcagc acagctccac acntgctttg ggccgcattt gcaagttctc 360
tgtaancccc ctgnaagacc cggatcagct gggtngaaat tgcangcnct cttttggca 419

<210> 14
<211> 400
<212> DNA
<213> Homo sapien

<220>
<221> misc_feature
<222> (1)...(400)
<223> n = A,T,C or G

<400> 14
aanccattgc caagggtatc cggaggattg tggctgtcac aggtnccgag gcccanaagg 60
ccctcaggaa agcaaagagc ttgaaaaatg tctctctgtc atggaagccn aagtgaaggc 120
tcanactgct ccaacaagga tntgcanagg gagatcgcta accttggaga ggccctggcc 180
actgcagtcn tcccccantg gcagaaggat gaattgcggg agactctcan atcccttang 240
gaaggtcgtg gatnacttgg accgagcctc nnaagccaat ntccagaaca agtgttggag 300
aagacaaagc anttcatcga cgccaacccc naccggcctc tnttctcctg ganattgana 360
gcggcgcccc cgcccagggc cttaataanc cntgaagctn 400

<210> 15
<211> 395
<212> DNA
<213> Homo sapien

<220>
<221> misc_feature
<222> (1)...(395)
<223> n = A,T,C or G

<400> 15
tgctttgctg cgtccaggaa gattagatng aanaatacat attgatttgc caaatgaaca 60
agcgagatta gacntactga anatccatgc aggtcccatt acaaagcatg gtgaaataga 120
tgatgaagca attgtgaagc tatcggatgg ctttnatgga gcagatctga gaaatgtttg 180
tactgaagca ggtatgttcg caattcgtgc tgatcatgat tttgtagtac aggaagactt 240
catgaaagcn gtcagaanag tggctnattc tnaaagctgg agtctaaatt ggacnacnac 300
ctntgtattt actgttggan ttttgatgct gcatgacaga ttttgcttan tgtaaaaatn 360
aagttcaaga aaattatgtt agttttggcc attat 395

<210> 16
<211> 404
<212> DNA
<213> Homo sapien

<220>
<221> misc_feature
<222> (1)...(404)
<223> n = A,T,C or G

<400> 16
ccaccactaa aatcctggct gagccctacn agtacctgtg cccctccccc aggacgagat 60
nagggcacac cctttaagtn aggtgacagg tcacctttaa gtgaggacag tcagctnaat 120
ttcacctctt gggcttgagt acctggttct cgtgccctga ggcgacnctn agccctgcag 180
ctnccatgta cgtgctgcca atngtcttga tcttctccac gccnctnaac ttgggcttca 240
gtaggagctg caggcnagaa ngaagcggtt aacagcgcca ctccatagcc gcagccnggc 300
tgcccctgct tctcaaggag gggtgtgggg ttcctccacc atcgccgccc ttgcaaacac 360
ntctcanggc ttccctnccg gctnancgca ngacttaagc atgg 404

<210> 17
<211> 360
<212> DNA
<213> Homo sapien

<220>
<221> misc_feature
<222> (1)...(360)
<223> n = A,T,C or G

<400> 17
ggccagaagc tttccacaaa ccagtgaagg tggcagcaaa gaaagcctct tagacnagga 60
gctggcagca gctgctatct ngatngacng cagaaaccaa ccactaattc agcaaacaca 120
acctcatacc tnaccgcttc cctttnaatg gccttcggtg tgtgcgcaca tgggcacgtg 180
cggggagaac catacttatt cccctnttcc cggcctacca cctctnctcc cccttctctt 240
ctctncaatt actntctccn ctgctttntt ctnancacta ctgctngtnt cnanagccng 300
cccgcaatta cctggcaaaa ctcgcgaccc ttcgggcagc gctaaanaat gcacatttac 360

<210> 18
<211> 316
<212> DNA
<213> Homo sapien

<220>
<221> misc_feature
<222> (1)...(316)
<223> n = A,T,C or G

<400> 18
atacatatac acatatatga ttttagatag agccatatac ctngaagtag tanatttgtt 60
tgtgtgtata tgtatgtgtc tactcatttt aaataaactt gtgatagaga tgtaattntg 120
agccagtttt tcatttgctt aaatnactca ccaagtaact aattaagttn tctttactct 180
taatgttnag tagtgagatt ctgttgaagg tgatattaaa aaccattcta tattaattaa 240
cattcatgtt gttttttaaa agcttatttg aaatcnaatt atgattattt ttcataccag 300
tcgatnttat gtangt 316

<210> 19
<211> 350
<212> DNA
<213> Homo sapien

<220>
<221> misc_feature
<222> (1)...(350)
<223> n = A,T,C or G

<400> 19
aagggatgca nataatgctg tgtatgagct tgatggaaaa gaactctgta gtgaaagggt 60
tactattgaa catgctnggg ctcggtcacg aggtggaaga ggtagaggac gatactctga 120
ccgttttagt agtcgcagac ctcgaaatga tagacgaaat gctccacctg taagaacaga 180
anatcgtctt atagttgaga atttatcctc aagagtcagc tggcaggttt gttganatac 240
agttttgagt tnttttgatg tggcttttta aaaaagttat gggttactna tgttatattg 300
ttttattaaa agtagttttn aattaatgga tntgatggaa ttgttgtttt 350

<210> 20
<211> 367
<212> DNA
<213> Homo sapien

<220>
<221> misc_feature
<222> (1)...(367)
<223> n = A,T,C or G

<400> 20
gntnnncnca agatcctnct ntcccccngg gcngccccnc cnccngtnat naccggtttn 60
ntaanatcnn gccgcncccg aagtctcnct nntgccgaga tgncccttat ncncnnatgn 120

ncaattntga cctnnggcga anaatggcng nngtgtatca gtntccnctc tgnggnctct 180
tagnatctga ccactangac ccnctatcct ctcaaaccct gtanncngcc ctaatttgtg 240
ccaattagtg catgntanag cntcctggcc cagatggcnt ccatatcctg gtncggcttc 300
cgcccctacc angncatccn catctactag agcttatccg ctncntgngg cgcaccggnt 360
ccccnct 367

<210> 21
<211> 366
<212> DNA
<213> Homo sapien

<220>
<221> misc_feature
<222> (1)...(366)
<223> n = A,T,C or G

<400> 21
cccaacacaa tggtctaagt anaactgtat tgctctgtag tatagttcca cattggcaac 60
ctacaatggg aaaatccata cataagtcag ttacttcctn atgagctttc tccttctgaa 120
tcctttatct tctgaagaaa gtacacacct tggtnatgat atctttgaat tgcccttctt 180
tccaggcatc agttggatga ttcatcatgg taattatggc attatcatat tcttcatact 240
tgtcatacga aaacaccagt tctgcccnna gatgagcttg ttctgcagct cttagcacct 300
tgggaatatt cactctagac cagaaacagc tcccggtgct ccctcatttt ctgaggctta 360
aatttn 366

<210> 22
<211> 315
<212> DNA
<213> Homo sapien

<220>
<221> misc_feature
<222> (1)...(315)
<223> n = A,T,C or G

<400> 22
acttaatgca atctctggag gataatttgg atcaagaaat aaagaanaaa tgaattagga 60
gaagaaatna ctgggtnata tttcaatatt ttagaacttt aanaatgttg actatgattt 120
caatatattt gtnaaaactg agatacangt ttgacctata tctgcatttt gataattaaa 180
cnaatnnatt ctatttnaat gttgtttcag agtcacagca cagactgaaa ctttttttga 240
atacctnaat atcacacttn tncttnnaat gatgttgaag acaatgatga catgccttna 300
gcatataatg tcgac 315

<210> 23
<211> 202
<212> DNA
<213> Homo sapien

<220>
<221> misc_feature
<222> (1)...(202)
<223> n = A,T,C or G

<400> 23
actaatccag tgtggtgnaa ttccattgtg ttgggcaact caggatatta aatttatnat 60
ttaaaaattc ccaagagaaa naaactccag gccctgattg tttcactggg gaattttacc 120
aaatgttnca nnaaganatg acgctgattc tgtnaaatct ttttcagaag atagaggaga 180
acacccaccg nttcatttta tg 202

<210> 24
<211> 365
<212> DNA
<213> Homo sapien

<220>
<221> misc_feature
<222> (1)...(365)
<223> n = A,T,C or G

<400> 24
   ggatttcttg cccttttctc cctttttaag tatcaatgta tgaaatccac ctgtaccacc 60
   ctttctgcca tacaaccgct accacatctg gctcctagaa cctgttttgc tttcatagat 120
   ggatctcgga accnagtgtt nacttcattt ttaaacccca ttttagcaga tngtttgctn 180
   tggtctgtct gtattcacca tggggcctgt acacaccacg tgtggttata gtcaaacaca 240
   gtgccctcca ttgtggccac atgggagacc catnacccna tactgcatcc tgggctgatn 300
   acggcactgc atctnacccg acntgggatt gaacccgggg tgggcagcng aattgaacag 360
   gatca 365

<210> 25
<211> 359
<212> DNA
<213> Homo sapien

<220>
<221> misc_feature
<222> (1)...(359)
<223> n = A,T,C or G

<400> 25
gtttcctgct tcaacagtgc ttggacggaa cccggcgctc gttccccacc ccggccggcc 60
gcccatagcc agccctccgt cacctcttca ccgcaccctc ggactgcccc aaggcccccg 120
ccgccnctcc ngcgccncgc agccaccgcc gccnccncca cctctccttn gtcccgccnt 180
nacaacgcgt ccacctcgca ngttcgccng aactaccacc nggactcata ngccgccctc 240
aaccgcccga tcaacctgga gctctncccc ccgacnttaa cctttccntg tcttacttac 300
nttaaccgcc gnttattttg cttnaaaaga acttttcccc aatactttct ttcaccnnt 359

<210> 26
<211> 400
<212> DNA
<213> Homo sapien

<220>
<221> misc_feature
<222> (1)...(400)
<223> n = A,T,C or G

<400> 26
agtgaaacag tatatgtgaa aaggagtttg tgannagcta cataaaaata ttagatatct 60
ttataatttc caataggata ctcatcagtt ttgaataana gacatattct agagaaacca 120
ggtttctggt ttcagatttg aactctcaag agcttggaag ttatcactcc catcctcacg 180
acnacnaana aatctnaacn aacngaanac caatgacttt tcttagatct gtcaaagaac 240
ttcagccacg aggaaaacta tcnccctnaa tactggggac tggaaagaga gggtacagag 300
aatcacagtg aatcatagcc caagatcagc ttgcccggag ctnaagctng tacgatnatt 360
acttacaggg accacttcac agtnngtnga tnaantgccn 400

<210> 27
<211> 366
<212> DNA
<213> Homo sapien

<220>
<221> misc_feature
<222> (1)...(366)
<223> n = A,T,C or G

<400> 27
gaatttctta gaaactgaag tttactctgt tccaagatat atcttcactg tcttaatcaa 60
agggcgctng aatcatagca aatattctca tctttcaact aactttaagt agttntcctg 120
gaattttaca ttttccagaa aacactcctt tctgtatctg tgaaagaaag tgtgcctcag 180
gctgtagact gggctgcact ggacacctgc gggggactct ggctnagtgn ggacatggtc 240
agtattgatt ttcctcanac tcagcctgtg tagctntgaa agcatggaac agattacact 300
gcagttnacg tcatcccaca catcttggac tccnagaccc ggggaggtca catagtccgt 360
tatgna 366

<210> 28
<211> 402

<212> DNA
<213> Homo sapien

<220>
<221> misc_feature
<222> (1)...(402)
<223> n = A,T,C or G

<400> 28
agtgggagcc tcctccttcc ccactcagtt ctttacatcc ccgaggcgca gctgggcnaa 60
ggaagtggcc agctgcagcg cctcctgcag gcagccaacg ttcttgcctg tggcctgtgc 120
agacacatcc ttgccaccac ctttaccgtc catcangcct gacacctgct gcacccactc 180
gctngctttt aagccccgat nggctgcatt ctgggggact tgacacaggc ncgtgatctt 240
gccagcctca ttgtccaccg tgaagagcat ggcaaaaagt ctgaggggag tgcatcttga 300
anagcttcaa ggcttcattc agggccttng ctnaggcgcc nctctccatc tccnggaata 360
acnagaggct ggtnngggtn actntcaata aactgcttcg tc 402

<210> 29
<211> 175
<212> DNA
<213> Homo sapien

<400> 29
cggacgggca tgaccggtcc ggtcagctgg gtggccagtt tcagttcttc agcagaactg 60
tctcccttct tgggggccga gggcttcctg gggaagagga tgagtttgga gcggtactcc 120
ttcagccgct gcacgttggt ctgcagggac tccgtggact tgttccgcct cctcg 175

<210> 30
<211> 360
<212> DNA
<213> Homo sapien

<400> 30
ttgtatttct tatgatctct gatgggttct tctcgaaaat gccaagtgga agactttgtg 60
gcatgctcca gatttaaatc cagctgaggc tccctttgtt ttcagttcca tgtaacaatc 120
tggaaggaaa cttcacggac aggaagactg ctggagaaga gaagcgtgtt agcccatttg 180
aggtctgggg aatcatgtaa agggtaccca gacctcactt ttagttattt acatcaatga 240
gttctttcag ggaaccaaac ccagaattcg gtgcaaaagc caaacatctt ggtgggattt 300
gataaatgcc ttgggacctg gagtgctggg cttgtgcaca ggaagagcac cagccgctga 360

<210> 31
<211> 380
<212> DNA
<213> Homo sapien

<220>
<221> misc_feature
<222> (1)...(380)
<223> n = A,T,C or G

<400> 31
acgctctaag cctgtccacg agctcaatag ggaagcctgt gatgactaca gactttgcga 60
acgctacgcc atggtttatg gatacaatgc tgcctataan cgctacttca ggaagcgccg 120
agggaccnaa tgagactgag ggaagaaaaa aaatctcttt ttttctggag gctggcacct 180
gattttgtat ccccctgtnn cagcattncn gaaatacata ggcttatata caatgcttct 240
ttcctgtata ttctcttgtc tggctgcacc ccttnttccc gcccccagat tgataagtaa 300
tgaaagtgca ctgcagtnag ggtcaangga gactcancat atgtgattgt tccntnataa 360
acttctggtg tgatactttc 380

<210> 32
<211> 440
<212> DNA
<213> Homo sapien

<220>
<221> misc_feature
<222> (1)...(440)
<223> n = A,T,C or G

<400> 32
gtgtatggga gcccctgact cctcacgtgc ctgatctgtg cccttggtcc caggtcaggc 60
ccaccccctg cacctccacc tgccccagcc cctgcctctg ccccaagtgg ggccagctgc 120
cctcacttct ggggtggatg atgtgacctt cctnggggga ctgcggaagg gacaagggtt 180
ccctgaagtc ttacggtcca acatcaggac caagtcccat ggacatgctg acagggtccc 240
caggggagac cgtntcanta gggatgtgtg cctggctgtg tacgtgggtg tgcagtgcac 300
gtganaagca cgtggcggct tctgggggcc atgtttgggg aaggaagtgt gcccnccacc 360
cttggagaac ctcagtcccn gtagccccct gccctggcac agcngcatnc acttcaaggg 420
caccctttgg gggttggggt 440

<210> 33
<211> 345
<212> DNA
<213> Homo sapien

<220>
<221> misc_feature
<222> (1)...(345)
<223> n = A,T,C or G

<400> 33
tattttaaca atgtttatta ttcatttatc cctctataga accaccaccc acaccgagga 60
gattatttgg agtgggtccc aacctagggc ctggactctg aaatctaact ccccacttcc 120
ctcattttgt gacttaggtg ggggcatggt tcagtcagaa ctggtgtctc ctattggatc 180
gtgcagaagg aggacctagg cacacacata tggtggccac acccaggagg gttgattggc 240
aggctggaag acaaaagtct cccaataaag gcacttttac ctcaaagang gggtgggagt 300
tggtctgctg ggaatgttgt tgttggggtg gggaagantt atttc 345

<210> 34
<211> 440
<212> DNA
<213> Homo sapien

<220>
<221> misc_feature
<222> (1)...(440)
<223> n = A,T,C or G

<400> 34
tgtaattttt ttattggaaa acaaatatac aacttggaat ggattttgag gcaaattgtg 60
ccataagcag attttaagtg gctaaacaaa gtttaaaaag caagtaacaa taaaagaaaa 120
tgtttctggt acaggaccag cagtacaaaa aaatagtgta cgagtacctg gataatacac 180
ccgttttgca atagtgcaac ttttaagtac atattgttga ctgtccatag tccacgcaga 240
gttacaactc cacacttcaa caacaacatg ctgacagttc ctaaagaaaa ctactttaaa 300
aaaggcataa cccagatgtt ccctcatttg accaactcca tctnagttta gatgtgcaga 360
agggcttana ttttcccaga gtaagccnca tgcaacatgt tacttgatca attttctaaa 420
ataaggtttt aggacaatga 440

<210> 35
<211> 540
<212> DNA
<213> Homo sapien

<220>
<221> misc_feature
<222> (1)...(540)
<223> n = A,T,C or G

<400> 35
atagatggaa tttattaagc ttttcacatg tgatagcaca tagttttaat tgcatccaaa 60
gtactaacaa aaactctagc aatcaagaat ggcagcatgt tattttataa caatcaacac 120
ctgtggcttt taaaatttgg ttttcataag ataatttata ctgaagtaaa tctagccatg 180
cttttaaaaa atgctttagg tcactccaag cttggcagtt aacatttggc ataaacaata 240
ataaaacaat cacaatttaa taaataacaa atacaacatt gtaggccata atcatataca 300
gtataaggga aaaggtggta gtgttganta agcagttatt agaatagaat accttggcct 360
ctatgcaaat atgtctagac actttgattc actcagccct gacattcagt tttcaaagtt 420
aggaaacagg ttctacagta tcattttaca gtttccaaca cattgaaaac aagtagaaaa 480

tgatganttg atttttatta atgcattaca tcctcaagan ttatcaccaa cccctcaggt 540

<210> 36
<211> 555
<212> DNA
<213> Homo sapien

<220>
<221> misc_feature
<222> (1)...(555)
<223> n = A,T,C or G

<400> 36
cttcgtgtgc ttgaaaattg gagcctgccc ctcggcccat aagcccttgt tgggaactga 60
gaagtgtata tggggcccaa nctactggtg ccagaacaca gagacagcag cccantgcaa 120
tgctgtcgag cattgcaaac gccatgtgtg gaactaggag gaggaatatt ccatcttggc 180
agaaaccaca gcattggttt ttttctactt gtgtgtctgg gggaatgaac gcacagatct 240
gtttgacttt gttataaaaa tagggctccc ccacctcccc cntttctgtg tnctttattg 300
tagcantgct gtctgcaagg gagcccctan cccctggcag acananctgc ttcagtgccc 360
ctttcctctc tgctaaatgg atgttgatgc actggaggtc ttttancctg cccttgcatg 420
gcncctgctg gaggaagana aaactctgct ggcatgaccc acagtttctt gactggangc 480
cntcaaccct cttggttgaa gccttgttct gaccctgaca tntgcttggg cnctgggtng 540
gnctgggctt ctnaa 555

<210> 37
<211> 280
<212> DNA
<213> Homo sapien

<220>
<221> misc_feature
<222> (1)...(280)
<223> n = A,T,C or G

<400> 37
ccaccgacta taagaactat gccctcgtgt attcctgtac ctgcatcatc caactttttc 60
acgtggattt tgcttggatc ttggcaagaa accctaatct ccctccagaa acagtggact 120
ctctaaaaaa tatcctgact tctaataaca ttgatntcaa gaaaatgacg gtcacagacc 180
aggtgaactg ccccnagctc tcgtaaccag gttctacagg gaggctgcac ccactccatg 240
ttncttctgc ttcgctttcc cctaccccac cccccgccat 280

<210> 38
<211> 303
<212> DNA
<213> Homo sapien

<220>
<221> misc_feature
<222> (1)...(303)
<223> n = A,T,C or G

<400> 38
catcgagctg gttgtcttct tgcctgccct gtgtcgtaaa atgggggtcc cttactgcat 60
tatcaaggga aaggcaagac tgggacgtct agtccacagg aagacctgca ccactgtcgc 120
cttcacacag gtgaactcgg aagacaaagg cgctttggct nagctggtgn aagctatcag 180
gaccaattac aatgacngat acgatnagat ccgccntcac tggggtagca atgtcctggg 240
tcctaagtct gtggctcgta tcgccnagct cgaanaggcn aangctaaag aacttgccac 300
taa 303

<210> 39
<211> 300
<212> DNA
<213> Homo sapien

<220>
<221> misc_feature
<222> (1)...(300)
<223> n = A,T,C or G

<400> 39
gactcagcgg ctggtgctct tcctgtgcac aagcccagca ctccaggtcc caaggcattt 60
atcaaatccc accaagatnt ttggcttttg caccgaattc tgggtttggt tccctnaaag 120
aactcattga tgtaaatnac tnaaagtgag gtctgggtac cctttacatg attccccaga 180
cctcanatgg gctaacacgc ttctcttctc cagcagtctt cctntccgtg aagttacctt 240
ccagattgtt acatggaact gaanacaaag ggagcctcag ctngatttaa atctggagca 300

<210> 40
<211> 318
<212> DNA
<213> Homo sapien

<220>
<221> misc_feature
<222> (1)...(318)
<223> n = A,T,C or G

<400> 40
cccaacacaa tggctgagga caaatcagtt ctctgtgacc agacatgaga aggttgccaa 60
tgggctgttg ggcgaccaag gccttcccgg agtcttcgtc ctctatgagc tctcgcccat 120
gatggtgaag ctgacggaga agcacaggtc cttcacccac ttcctgacag gtgtgtgcgc 180
catcattggg ggcatgttca cagtggctgg actcatcgat tcgctcatct accactcagc 240
acgagccatc cagaaaaaaa ttgatctngg gaagacnacg tagtcaccct cggtncttcc 300
tctgtctcct ctttctcc 318

<210> 41
<211> 302
<212> DNA
<213> Homo sapien

<220>
<221> misc_feature
<222> (1)...(302)
<223> n = A,T,C or G

<400> 41
acttagatgg ggtccgttca ggggatacca gcgttcacat tttccttttt aagaaagggt 60
cttggcctga atgttcccca tccggacaca ggctgcatgt ctctgtnagt gtcaaagctg 120
ccatnaccat ctcggtaacc tactcttact ccacaatgtc tatnttcact gcagggctct 180
ataatnagtc cataatgtaa atgcctggcc caagacntat ggcctgagtt tatccnaggc 240
ccaaacnatt accagacatt cctcttanat tgaaaacgga tntctttccc ttggcaaaga 300
tc 302

<210> 42
<211> 299
<212> DNA
<213> Homo sapien

<220>
<221> misc_feature
<222> (1)...(299)
<223> n = A,T,C or G

<400> 42
cttaataagt ttaaggccaa ggcccgttcc attcttctag caactgacgt tgccagccga 60
ggtttggaca tacctcatgt aaatgtggtt gtcaactttg acattcctac ccattccaag 120
gattacatcc atcgagtagg tcgaacagct agagctgggc gctccggaaa ggctattact 180
tttgtcacac agtatgatgt ggaactcttc cagcgcatag aacacttnat tgggaagaaa 240
ctaccaggtt ttccaacaca ggatgatgag gttatgatgc tnacggaacg cgtcgctna 299

<210> 43
<211> 305
<212> DNA
<213> Homo sapien

<220>
<221> misc_feature

<222> (1)...(305)
<223> n = A,T,C or G

<400> 43
ccaacaatgt caagacagcc gtctgtgaca tcccacctcg tggcctcaan atggcagtca 60
ccttcattgg caatagcaca gccntccggg agctcttcaa gcgcatctcg gagcagttca 120
ctgccatgtt ccgccggaag gccttcctcc actggtacac aggcgagggc atggacaaga 180
tggagttcac cgaggctgag agcaacatga acgacctcgt ctctnagtat cagcagtacc 240
gggatgccac cgcagaaana ggaggaggat ttcggtnagg aggccgaaga aggaggcctg 300
aggca 305

<210> 44
<211> 399
<212> DNA
<213> Homo sapien

<220>
<221> misc_feature
<222> (1)...(399)
<223> n = A,T,C or G

<400> 44
tttctgtggg ggaaacctga tctcgacnaa attagagaat tttgtcagcg gtatttcggc 60
tggaacagaa cgaaaacnga tnaatctctg tttcctgtat taaagcaact cgatncccag 120
cagacacagc tccnaattga ttccttcttt ngattagcac aacagggaga aagaanatgc 180
ttaacgtatt aagagccnga gactaaacag agctttgaca tgtatgctta ggaaagagaa 240
agaagcagcn gcccgcgnaa ttngaagcng tttctgttgc cntgganaaa gaatttgagc 300
ttctttatta ggccaacgaa aaaccccgaa ananaggcnt tacnatacct tngaaaantc 360
tccngccnna aaaagaaaga agctttcnga ttcttaacc 399

<210> 45
<211> 440
<212> DNA
<213> Homo sapien

<220>
<221> misc_feature
<222> (1)...(440)
<223> n = A,T,C or G

<400> 45
gcgggagcag aagctaaagc caaagcccaa gagagtggca gtgccagcac tggtgccagt 60
accagtacca ataacagtgc cagtgccagt gccagcacca gtggtggctt cagtgctggt 120
gccagcctga ccgccactct cacatttggg ctcttcgctg gccttggtgg agctggtgcc 180
agcaccagtg gcagctctgg tgcctgtggt ttctcctaca agtgagattt taggtatctg 240
ccttggtttc agtggggaca tctggggctt anggggcngg gataaggagc tggatgattc 300
taggaaggcc cangttggag aangatgtgn anagtgtgcc aagacactgc ttttggcatt 360
ttattccttt ctgtttgctg gangtcaatt gacccttnna ntttctctta cttgtgtttt 420
canatatngt taatcctgcc 440

<210> 46
<211> 472
<212> DNA
<213> Homo sapien

<220>
<221> misc_feature
<222> (1)...(472)
<223> n = A,T,C or G

<400> 46
gctctgtaat ttcacatttt aaaccttccc ttgacctcac attcctcttc ggccacctct 60
gtttctctgt tcctcttcac agcaaaaact gttcaaaaga gttgttgatt actttcattt 120
ccactttctc acccccattc tcccctcaat taactctcct tcatccccat gatgccatta 180
tgtggctntt attanagtca ccaaccttat tctccaaaac anaagcaaca aggactttga 240
cttctcagca gcactcagct ctggtncttg aaacaccccc gttacttgct attcctccta 300
cctcataaca atctccttcc cagcctctac tgctgccttc tctgagttct tcccagggtc 360
ctaggctcag atgtagtgta gctcaaccct gctacacaaa gnaatctcct gaaagcctgt 420

aaaaatgtcc atncntgtcc tgtgagtgat ctnccangna naataacaaa tt 472

<210> 47
<211> 550
<212> DNA
<213> Homo sapien

<220>
<221> misc_feature
<222> (1)...(550)
<223> n = A,T,C or G

<400> 47
ccttcctccg cctggccatc cccagcatgc tcatgctgtg catggagtgg tgggcctatg 60
aggtcgggag cttcctcagt ggtctgtatg aggatggatg acggggactg gtgggaacct 120
gggggccctg tctgggtgca aggcgacagc tgtctttctt caccaggcat cctcggcatg 180
gtggagctgg gcgctcagtc catcgtgtat gaactggcca tcattgtgta catggtccct 240
gcaggcttca gtgtggctgc cagtgtccgg gtangaaacg ctctgggtgc tggagacatg 300
gaagcaggca cggaagtcct ctaccgtttc cctgctgatt acagtgctct ttgctgtanc 360
cttcagtgtc ctgctgttaa gctgtaagga tcacntgggg tacattttta ctaccgaccg 420
agaacatcat taatctggtg gctcaggtgg ttccaattta tgctgtttcc cacctctttg 480
aagctcttgc tgctcaggta cacgccaatt ttgaaaagta aacaacgtgc ctcggagtgg 540
gaattctgct 550

<210> 48
<211> 214
<212> DNA
<213> Homo sapien

<220>
<221> misc_feature
<222> (1)...(214)
<223> n = A,T,C or G

<400> 48
agaaggacat aaacaagctg aacctgccca agacgtgtga tatcagcttc tcagatccag 60
acaacctcct caacttcaag ctggtcatct gtcctgatna gggcttctac nagagtggga 120
agtttgtgtt cagttttaag gtgggccagg gttacccgca tgatcccccc aaggtgaagt 180
gtgagacnat ggtctatcac cccnacattg acct 214

<210> 49
<211> 267
<212> DNA
<213> Homo sapien

<220>
<221> misc_feature
<222> (1)...(267)
<223> n = A,T,C or G

<400> 49
atctgcctaa aatttattca aataatgaaa atnaatctgt tttaagaaat tcagtctttt 60
agtttttagg acaactatgc acaaatgtac gatggagaat tctttttgga tnaactctag 120
gtngaggaac ttaatccaac cggagctntt gtgaaggtca gaanacagga gagggaatct 180
tggcaaggaa tggagacnga gtttgcaaat tgcagctaga gtnaatngtt ntaaatggga 240
ctgctnttgt gtctcccang gaaagtt 267

<210> 50
<211> 300
<212> DNA
<213> Homo sapien

<220>
<221> misc_feature
<222> (1)...(300)
<223> n = A,T,C or G

<400> 50

gactgggtca aagctgcatg aaaccaggcc ctggcagcaa cctgggaatg gctggaggtg 60
ggagagaacc tgacttctct ttccctctcc ctcctccaac attactggaa ctctgtcctg 120
ttgggatctt ctgagcttgt ttccctgctg ggtgggacag aggacaaagg agaagggagg 180
gtctagaaga ggcagccctt ctttgtcctc tggggtnaat gagcttgacc tanagtagat 240
ggagagacca anagcctctg atttttaatt tccataanat gttcnaagta tatntntacc 300

<210> 51
<211> 300
<212> DNA
<213> Homo sapien

<220>
<221> misc_feature
<222> (1)...(300)
<223> n = A,T,C or G

<400> 51
gggtaaaatc ctgcagcacc cactctggaa aatactgctc ttaattttcc tgaaggtggc 60
cccctatttc tagttggtcc aggattaggg atgtggggta tagggcattt aaatcctctc 120
aagcgctctc caagcacccc cggcctgggg gtnagtttct catcccgcta ctgctgctgg 180
gatcaggttn aataaatgga actcttcctg tctggcctcc aaagcagcct aaaaactgag 240
gggctctgtt agaggggacc tccaccctnn ggaagtccga ggggctnggg aagggtttct 300

<210> 52
<211> 267
<212> DNA
<213> Homo sapien

<220>
<221> misc_feature
<222> (1)...(267)
<223> n = A,T,C or G

<400> 52
aaaatcaact tcntgcatta atanacanat tctanancag gaagtgaana taattttctg 60
cacctatcaa ggaacnnact tgattgcctc tattnaacan atatatcgag ttnctatact 120
tacctgaata ccnccgcata actctcaacc nanatncntc nccatgacac tcnttcttna 180
atgctantcc cgaattcttc attatatcng tgatgttcgn cctgntnata tatcagcaag 240
gtatgtnccn taactgccga nncaang 267

<210> 53
<211> 401
<212> DNA
<213> Homo sapien

<400> 53
agsctttagc atcatgtaga agcaaactgc acctatggct gagataggtg caatgaccta 60
caagattttg tgttttctag ctgtccagga aaagccatct tcagtcttgc tgacagtcaa 120
agagcaagtg aaaccatttc cagcctaaac tacataaaag cagccgaacc aatgattaaa 180
gacctctaag gctccataat catcattaaa tatgcccaaa ctcattgtga ctttttattt 240
tatatacagg attaaaatca acattaaatc atcttattta catggccatc ggtgctgaaa 300
ttgagcattt taaatagtac agtaggctgg tatacattag gaaatggact gcactggagg 360
caaatagaaa actaaagaaa ttagataggc tggaaatgct t 401

<210> 54
<211> 401
<212> DNA
<213> Homo sapien

<400> 54
cccaacacaa tggataaaaa cacttatagt aaatggggac attcactata atgatctaag 60
aagctacaga ttgtcatagt tgttttcctg ctttacaaaa ttgctccaga tctggaatgc 120
cagtttgacc tttgtcttct ataatatttc ctttttttcc cctctttgaa tctctgtata 180
tttgattctt aactaaaatt gttctcttaa atattctgaa tcctggtaat taaaagtttg 240
ggtgtatttt ctttacctcc aaggaaagaa ctactagcta caaaaaatat tttggaataa 300
gcattgtttt ggtataaggt acatattttg gttgaagaca ccagactgaa gtaaacagct 360
gtgcatccaa tttattatag ttttgtaagt aacaatatgt a 401

<210> 55
<211> 933
<212> DNA
<213> Homo sapien

<400> 55
   tttactgctt ggcaaagtac cctgagcatc agcagagatg ccgagatgaa atcagggaac 60
   tcctagggga tgggtcttct attacctggg aacacctgag ccagatgcct tacaccacga 120
   tgtgcatcaa ggaatgcctc cgcctctacg caccggtagt aaactatccc ggttactcga 180
   caaacccatc acctttccag atggacgctc cttacctgca ggaataactg tgtttatcaa 240
   tatttgggct cttcaccaca acccctattt ctgggaagac cctcaggtct ttaacccctt 300
   gagattctcc agggaaaatt ctgaaaaaat acatccctat gccttcatac cattctcagc 360
   tggattaagg aactgcattg ggcagcattt tgccataatt gagtgtaaag tggcagtggc 420
   attaactctg ctccgcttca agctggctcc agaccactca aggccaccca gctgtcgtca 480
   agttgcctca agtccaagaa tggaatccat gtgtttgcaa aaaaagtttg ctaattttaa 540
   gtccttttcg tataagaatt aakgagacaa ttttcctacc aaaggaagaa caaaaggata 600
   aatataatac aaaatatatg tatatggttg tttgacaaat tatataactt aggatacttc 660
   tgactggttt tgacatccat taacagtaat tttaatttct ttgctgtatc tggtgaaacc 720
   cacaaaaaca cctgaaaaaa ctcaagctga gttccaatgc gaagggaaat gattggtttg 780
   ggtaactagt ggtagagtgg ctttcaagca tagtttgatc aaaactccac tcagtatctg 840
   cattactttt atctctgcaa atatctgcat gatagcttta ttctcagtta tctttcccca 900
   taataaaaaa tatctgccaa aaaaaaaaaa aaa 933

<210> 56
<211> 480
<212> DNA
<213> Homo sapien

<400> 56
ggctttgaag catttttgtc tgtgctccct gatcttcagg tcaccaccat gaagttctta 60
gcagtcctgg tactcttggg agtttccatc tttctggtct ctgcccagaa tccgacaaca 120
gctgctccag ctgacacgta tccagctact ggtcctgctg atgatgaagc ccctgatgct 180
gaaaccactg ctgctgcaac cactgcgacc actgctgctc ctaccactgc aaccaccgct 240
gcttctacca ctgctcgtaa agacattcca gttttaccca aatgggttgg ggatctcccg 300
aatggtagag tgtgtccctg agatggaatc agcttgagtc ttctgcaatt ggtcacaact 360
attcatgctt cctgtgattt catccaacta cttaccttgc ctacgatatc ccctttatct 420
ctaatcagtt tattttcttt caaataaaaa ataactatga gcaacaaaaa aaaaaaaaaa 480

<210> 57
<211> 798
<212> DNA
<213> Homo sapien

<400> 57
agcctacctg gaaagccaac cagtcctcat aatggacaag atccaccagc tcctcctgtg 60
gactaacttt gtgatatggg aagtgaaaat agttaacacc ttgcacgacc aaacgaacga 120
agatgaccag agtactctta accccttaga actgtttttc cttttgtatc tgcaatatgg 180
gatggtattg ttttcatgag cttctagaaa tttcacttgc aagtttattt ttgcttcctg 240
tgttactgcc attcctattt acagtatatt tgagtgaatg attatatttt taaaaagtta 300
catggggctt ttttggttgt cctaaactta caaacattcc actcattctg tttgtaactg 360
tgattataat ttttgtgata atttctggcc tgattgaagg aaatttgaga ggtctgcatt 420
tatatatttt aaatagattt gataggtttt taaattgctt tttttcataa ggtatttata 480
aagttatttg ggttgtctg ggattgtgtg aaagaaaatt agaaccccgc tgtatttaca 540
tttaccttgg tagtttattt gtggatggca gttttctgta gttttgggga ctgtggtagc 600
tcttggattg ttttgcaaat tacagctgaa atctgtgtca tggattaaac tggcttatgt 660
ggctagaata ggaagagaga aaaaatgaaa tggttgttta ctaattttat actcccatta 720
aaaattttta atgttaagaa aaccttaaat aaacatgatt gatcaatatg gaaaaaaaaa 780
aaaaaaaaaa aaaaaaaa 798

<210> 58
<211> 280
<212> DNA
<213> Homo sapien

<400> 58
ggggcagctc ctgaccctcc acagccacct ggtcagccac cagctggggc aacgagggtg 60
gaggtcccac tgagcctctc gcctgccccc gccactcgtc tggtgcttgt tgatccaagt 120
cccctgcctg gtcccccaca aggactccca tccaggcccc ctctgccctg ccccttgtca 180

tggaccatgg tcgtgaggaa gggctcatgc cccttattta tgggaaccat ttcattctaa 240
cagaataaac cgagaaggaa accagaaaaa aaaaaaaaaa 280

<210> 59
<211> 382
<212> DNA
<213> Homo sapien

<400> 59
aggcgggagc agaagctaaa gccaaagccc aagagagtgg cagtgccagc actggtgcca 60
gtaccagtac caataacagt gccagtgcca gtgccagcac cagtggtggc ttcagtgctg 120
gtgccagcct gaccgccact ctcacatttg ggctcttcgc tggccttggt ggagctggtg 180
ccagcaccag tggcagctct ggtgcctgtg gtttctccta caagtgagat tttagatatt 240
gttaatcctg ccagtctttc tcttcaagcc agggtgcatc ctcagaaacc tactcaacac 300
agcactctag gcagccacta tcaatcaatt gaagttgaca ctctgcatta aatctatttg 360
ccatttaaaa aaaaaaaaaa aa 382

<210> 60
<211> 602
<212> DNA
<213> Homo sapien

<400> 60
tgaagagccg cgcggtggag ctgctgcccg atgggactgc caaccttgcc aagctgcagc 60
ttgtggtgga gaatagtgcc cagcgggtca tccacttggc gggtcagtgg gagaagcacc 120
gggtcccatc ctcgtgagta ccgccactcc gaaagctgca ggattgcaga gagctggaat 180
cttctcgacg gctggcagag atccaagaac tgcaccagag tgtccgggcg gctgctgaag 240
aggcccgcag gaaggaggag gtctataagc agctgatgtc agagctggag actctgccca 300
gagatgtgtc ccggctggcc tacacccagc gcatcctgga gatcgtgggc aacatccgga 360
agcagaagga agagatcacc aagatcttgt ctgatacgaa ggagcttcag aaggaaatca 420
actccctatc tgggaagctg gaccggacgt ttgcggtgac tgatgagctt gtgttcaagg 480
atgccaagaa ggacgatgct gttcggaagg cctataagta tctagctgct ctgcacgaga 540
actgcagcca gctcatccag accatcgagg acacaggcac catcatgcgg gaggttcgag 600
ac 602

<210> 61
<211> 1368
<212> DNA
<213> Homo sapien

<220>
<221> misc_feature
<222> (1)...(1368)
<223> n = A,T,C or G

<400> 61
ccagtgagcg cgcgtaatac gactcactat agggcgaatt gggtaccggg ccccccctcg 60
agcggccgcc cttttttttt tttttttatt gatcagaatt caggctttat tattgagcaa 120
tgaaaacagc taaaacttaa ttccaagcat gtgtagttaa agtttgcaaa gtgggatatt 180
gttcacaaaa cacattcaat gtttaaacac tatttatttg aagaacaaaa tatatttaaa 240
attgtttgct tctaaaaagc ccatttccct ccaagtctaa actttgtaat ttgatattaa 300
gcaatgaagt tattttgtac aatctagtta aacaagcaga atagcactag gcagaataaa 360
aaattgcaca gacgtatgca attttccaag atagcattct ttaaattcag ttttcagctt 420
ccaaagattg gttgcccata atagacttaa acatataatg atggctaaaa aaaataagta 480
tacgaaaatg taaaaaagga aatgtaagtc cactctcaat ctcataaaag gtgagagtaa 540
ggatgctaaa gcaaaataaa tgtaggttct ttttttctgt ttccgtttat catgcaatct 600
gcttctttga tatgccttag ggttacccat ttaagttaga ggttgtaatg caatggtggg 660
aatgaaaatt gatcaaatat acaccttgtc atttcatttc aaattgcggg ctggaaactt 720
ccaaaaaaag ggtaggcatg aagaaaaaaa aaatcmaatc agaacctctt caggggtttg 780
kgktctgata tggcagacar gatacaagtc ccaccaggag atggagcaat tcaaaataag 840
ggtaatgggc tgacaaggta ttattgccag catgggacag aatgagcaac aggctgaaaa 900
gtttttggat tatatagcac ctagagtctc tgatgtaggg aatttttgtt agtcaaacat 960
acgctaaact tccaagggaa aatctttcag gtagcctaag cttgcttttc tagagtgatg 1020
agttgcattg ctactgtgat tttttgaaaa caaactgggt ttgtacaagt gagaaagact 1080
agagagaaag attttagtct gtttagcaga agccatttta tctgcgtgca catggatcaa 1140
tatttctgat cccctatacc ccaggaaggg caaaatccca aagaaatgtg ttagcaaaat 1200
tggctgatgc tatcatattg ctatggacat tgatcttgcc caacacaatg gaattccacc 1260
acactggact agtggatcca ctagttctag agcggccggc caccgcggtg gagctccagc 1320

ttttgttccc tttagtgagg gttaattgcg cgcttggcgt aatcatnn 1368

<210> 62
<211> 924
<212> DNA
<213> Homo sapien

<220>
<221> misc_feature
<222> (1)...(924)
<223> n = A,T,C or G

<400> 62
caaaggnaca ggaacagctt gnaaagtact gncatncctn cctgcaggga ccagcccttt 60
gcctccaaaa gcaataggaa atttaaaaga tttncactga gaaggggncc acgtttnart 120
tntnaatgtn tcargnanar tnccttncaa atgncrnctn cactnactnr gnatttgggt 180
tnccgnrtnc mgnactatnt caggtttgaa aaactggatc tgccacttat cagttatgtg 240
accttaaaga actccgttaa tttctcagag cctcagtttc cttgtctata agttgggagt 300
aatattaata ctatcatttt tccaaggatt gatgtgaaca ttaatgaggt gaaatgacag 360
atgtgtatca tggttcctaa taaacatcca aaatatagta cttactattg tcattattat 420
tacttgtttg aagctaaaga cctcacaata gaatcccatc cagcccacca gacagagytc 480
tgagttttct agtttggaag agctattaaa taacaacktc tagtgtcaat tctatacttg 540
ttatggtcaa gtaactgggc tcagcatttt acattcattg tctctttaag ttctagcaat 600
gtgaagcagg aactatgatt atattgacta cataaatgaa gaaattgagg ctcagataca 660
ttaagtaatt ctcccagggt cacacagcta gaactggcaa agcctgggat tgatccatga 720
tcttccagca ttgaagaatc ataaatgtaa ataactgcaa ggccttttcc tcagaagagc 780
tcctggtgct tgcaccaacc cactagcact tgttctctac aggggaacat ctgtgggcct 840
gggaatcact gcacgtcgca agagatgttg cttctgatga attattgttc ctgtcagtgg 900
tgtgaaggca aaaaaaaaaa aaaa 924

<210> 63
<211> 1079
<212> DNA
<213> Homo sapien

<400> 63
agtcccaaga actcaataat ctcttatgtt ttcttttgaa gacttatttt aaatattaac 60
tatttcggtg cctgaatgga aaaatataaa cattagctca gagacaatgg ggtacctgtt 120
tggaatccag ctggcagcta taagcaccgt tgaaaactct gacaggcttt gtgccctttt 180
tattaaatgg cctcacatcc tgaatgcagg aatgtgttcg tttaaataaa cattaatctt 240
taatgttgaa ttctgaaaac acaaccataa atcatagttg gtttttctgt gacaatgatc 300
tagtacatta tttcctccac agcaaaccta cctttccaga aggtggaaat tgtatttgca 360
acaatcaggg caaaacccac acttgaaaag cattttacaa tattatatct aagttgcaca 420
gaagacccca gtgatcacta ggaaatctac cacagtccag tttttctaat ccaagaaggt 480
caaacttcgg ggaataatgt gtccctcttc tgctgctgct ctgaaaaata ttcgatcaaa 540
acgaagttta caagcagcag ttattccaag attagagttc atttgtgtat cccatgtata 600
ctggcaatgt ttaggtttgc ccaaaaactc ccagacatcc acaatgttgt tgggtaaacc 660
accacatctg gtaacctctc gatcccttag atttgtatct cctgcaaata taactgtagc 720
tgactctgga gcctcttgca ttttctttaa aaccattttt aactgattca ttcgttccgc 780
agcatgccct ctggtgctct ccaaatggga tgtcataagg caaagctcat ttcctgacac 840
attcacatgc acacataaaa ggtttctcat cattttggta cttggaaaag gaataatctc 900
ttggcttttt aatttcactc ttgatttctt caacattata gctgtgaaat atccttcttc 960
atgacctgta ataatctcat aattacttga tctcttcttt aggtagctat aatatggggg 1020
aataacttcc tgtagaaata tcacatctgg gctgtacaaa gctaagtagg aacacaccc 1079

<210> 64
<211> 1001
<212> DNA
<213> Homo sapien

<400> 64
gaatgtgcaa cgatcaagtc agggtatctg tggtatccac cactttgagc atttatcgat 60
tctatatgtc aggaacattt caagttatct gttctagcaa ggaaatataa aatacttata 120
gttaactatg gcctatctac agtgcaacta aaaactagat tttattcctt tccacctgtg 180
ggtttgtatt catttaccac cctcttttca ttccctttct cacccacaca ctgtgccggg 240
cctcaggcat atactattct actgtctgtc tctgtaagga ttatcatttt agcttccaca 300
tatgagagaa tgcatgcaaa gtttttcttt ccatgtctgg cttatttcac ttaacataat 360
gacctccgct tccatccatg ttatttatat tacccaatag tgttcataaa tatatataca 420

cacatatata ccacattgca tttgtccaat tattcattga cggaaactgg ttaatgttat 480
atcgttgcta ttgtggatag tgctgcaata aacacgcaag tggggatata atttgaagag 540
tttttttgtt gatgttcctc caaattttaa gattgttttg tctatgtttg tgaaaatggc 600
gttagtattt tcatagagat tgcattgaat ctgtagattg ctttgggtaa gtatggttat 660
tttgatggta ttaatttttt cattccatga agatgagatg tctttccatt gtttgtgtcc 720
tctacatttt ctttcatcaa agttttgttg tatttttgaa gtagatgtat ttcaccttat 780
agatcaagtg tattccctaa atattttatt tttgtagcta ttgtagatga aattgccttc 840
ttgatttctt tttcacttaa ttcattatta gtgtatggaa atgttatgga tttttatttg 900
ttggttttta atcaaaaact gtattaaact tagagttttt tgtggagttt ttaagttttt 960
ctagatataa gatcatgaca tctaccaaaa aaaaaaaaaa a 1001

<210> 65
<211> 575
<212> DNA
<213> Homo sapien

<400> 65
acttgatata aaaaggatat ccataatgaa tattttatac tgcatccttt acattagcca 60
ctaaatacgt tattgcttga tgaagacctt tcacagaatc ctatggattg cagcatttca 120
cttggctact tcatacccat gccttaaaga ggggcagttt ctcaaaagca gaaacatgcc 180
gccagttctc aagttttcct cctaactcca tttgaatgta agggcagctg gcccccaatg 240
tggggaggtc cgaacatttt ctgaattccc attttcttgt tcgcggctaa atgacagttt 300
ctgtcattac ttagattccc gatctttccc aaaggtgttg atttacaaag aggccagcta 360
atagccagaa atcatgaccc tgaaagagag atgaaatttc aagctgtgag ccaggcagga 420
gctccagtat ggcaaaggtt cttgagaatc agccatttgg tacaaaaaag atttttaaag 480
cttttatgtt ataccatgga gccatagaaa ggctatggat tgtttaagaa ctattttaaa 540
gtgttccaga cccaaaaagg aaaaaaaaaa aaaaa 575

<210> 66
<211> 831
<212> DNA
<213> Homo sapien

<400> 66
attgggctcc ttctgctaaa cagccacatt gaaatggttt aaaagcaagt cagatcaggt 60
gatttgtaaa attgtattta tctgtacatg tatgggcttt taattcccac caagaaagag 120
agaaattatc tttttagtta aaaccaaatt tcacttttca aaatatcttc caacttattt 180
attggttgtc actcaattgc ctatatatat atatatatat gtgtgtgtgt gtgtgtgcgc 240
gtgagcgcac gtgtgtgtat gcgtgcgcat gtgtgtgtat gtgtattatc agacataggt 300
ttctaacttt tagatagaag aggagcaaca tctatgccaa atactgtgca ttctacaatg 360
gtgctaatct cagacctaaa tgatactcca tttaatttaa aaaagagttt taaataatta 420
tctatgtgcc tgtatttccc ttttgagtgc tgcacaacat gttaacatat tagtgtaaaa 480
gcagatgaaa caaccacgtg ttctaaagtc tagggattgt gctataatcc ctatttagtt 540
caaaattaac cagaattctt ccatgtgaaa tggaccaaac tcatattatt gttatgtaaa 600
tacagagttt taatgcagta tgacatccca caggggaaaa gaatgtctgt agtgggtgac 660
tgttatcaaa tattttatag aatacaatga acggtgaaca gactggtaac ttgtttgagt 720
tcccatgaca gatttgagac ttgtcaatag caaatcattt ttgtatttaa atttttgtac 780
tgatttgaaa aacatcatta aatatcttta aaagtaaaaa aaaaaaaaaa a 831

<210> 67
<211> 590
<212> DNA
<213> Homo sapien

<400> 67
gtgctctgtg tattttttta ctgcattaga cattgaatag taatttgcgt taagatacgc 60
ttaaaggctc tttgtgacca tgtttccctt tgtagcaata aaatgttttt tacgaaaact 120
ttctccctgg attagcagtt taaatgaaac agagttcatc aatgaaatga gtatttaaaa 180
taaaaatttg ccttaatgta tcagttcagc tcacaagtat tttaagatga ttgagaagac 240
ttgaattaaa gaaaaaaaaa ttctcaatca tatttttaaa atataagact aaaattgttt 300
ttaaaacaca tttcaaatag aagtgagttt gaactgacct tatttatact ctttttaagt 360
ttgttccttt tccctgtgcc tgtgtcaaat cttcaagtct tgctgaaaat acatttgata 420
caaagttttc tgtagttgtg ttagttcttt tgtcatgtct gtttttggct gaagaaccaa 480
gaagcagact tttcttttaa aagaattatt tctctttcaa atatttctat cctttttaaa 540
aaattccttt ttatggctta tatacctaca tatttaaaaa aaaaaaaaaa 590

<210> 68
<211> 301

<212> DNA
<213> Homo sapien

<220>
<221> misc_feature
<222> (1)...(301)
<223> n = A,T,C or G

<400> 68
ttgtgttggg gttccctttt ccggtcggcg tggtcttgcg agtggagtgt ccgctgtgcc 60
cgggcctgca ccatgagcgt cccggccttc atcgacatca gtgaagaaga tcaggctgct 120
gagcttcgtg cttatctgaa atctaaagga gctgagattt cagaagagaa ctcggaaggt 180
ggacttcatg ttgatttagc tcaaattatt gaagcctgtg atgtgtgtct gaaggaggat 240
gataaagatg ttgaaagtgt gatgaacagt ggggnatcct actcttgatc cggaanccna 300
c 301

<210> 69
<211> 301
<212> DNA
<213> Homo sapien

<220>
<221> misc_feature
<222> (1)...(301)
<223> n = A,T,C or G

<400> 69
tctatgagca tgccaaggct ctgtgggagg atgaaggagt gcgtgcctgc tacgaacgct 60
ccaacgagta ccagctgatt gactgtgccc agtacttcct ggacaagatc gacgtgatca 120
agcaggctga ctatgtgccg agcgatcagg acctgcttcg ctgccgtgtc ctgacttctg 180
gaatctttga gaccaagttc caggtggacn aagtcaactt ccacatgntt gacgtgggtg 240
gccagcgcga tgaacgccgc aagtggatcc agtgcttcaa cgatgtgact gccatcatct 300
t 301

<210> 70
<211> 201
<212> DNA
<213> Homo sapien

<400> 70
gcggctcttc ctcgggcagc ggaagcggcg cggcggtcgg agaagtggcc taaaacttcg 60
gcgttgggtg aaagaaaatg gcccgaacca agcagactgc tcgtaagtcc accggtggga 120
aagccccccg caaacagctg gccacgaaag ccgccaggaa aagcgctccc tctaccggcg 180
gggtgaagaa gcctcatcgc t 201

<210> 71
<211> 301
<212> DNA
<213> Homo sapien

<220>
<221> misc_feature
<222> (1)...(301)
<223> n = A,T,C or G

<400> 71
gccggggtag tcgccgncgc cgccgccgct gcagccactg caggcaccgc tgccgccgcc 60
tgagtagtgg gcttaggaag gaagaggtca tctcgctcgg agcttcgctc ggaagggtct 120
ttgttccctg cagccctccc acgggaatga caatggataa aagtgagctg gtacanaaag 180
ccaaactcgc tgagcaggct gagcgatatg atgatatggc tgcagccatg aaggcagtca 240
cagaacaggg gcatgaactc ttcaacgaag agagaaatct gctctctggt gcctacaaga 300
a 301

<210> 72
<211> 251
<212> DNA
<213> Homo sapien

<220>
<221> misc_feature
<222> (1)...(251)
<223> n = A,T,C or G

<400> 72
cttggggggt gttgggggag agactgtggg cctggaaata aaacttgtct cctctaccac 60
caccctgtac cctagcctgc acctgtccac atctctgcaa agttcagctt ccttccccag 120
gtctctgtgc actctgtctt ggatgctctg gggagctcat gggtggagga gtctccacca 180
gagggaggct caggggactg gttgggccag ggatgaatat ttgagggata aaaattgtgt 240
aagagccaan g 251

<210> 73
<211> 913
<212> DNA
<213> Homo sapien

<400> 73
tttttttttt tttttcccag gccctctttt tatttacagt gataccaaac catccacttg 60
caaattcttt ggtctcccat cagctggaat taagtaggta ctgtgtatct ttgagatcat 120
gtatttgtct ccactttggt ggatacaaga aaggaaggca cgaacagctg aaaaagaagg 180
gtatcacacc gctccagctg gaatccagca ggaacctctg agcatgccac agctgaacac 240
ttaaaagagg aaagaaggac agctgctctt catttatttt gaaagcaaat tcatttgaaa 300
gtgcataaat ggtcatcata agtcaaacgt atcaattaga ccttcaacct aggaaacaaa 360
attttttttt tctatttaat aatacaccac actgaaatta tttgccaatg aatcccaaag 420
atttggtaca aatagtacaa ttcgtatttg ctttcctctt tcctttcttc agacaaacac 480
caaataaaat gcaggtgaaa gagatgaacc acgactagag gctgacttag aaatttatgc 540
tgactcgatc taaaaaaaat tatgttggtt aatgttaatc tatctaaaat agagcatttt 600
gggaatgctt ttcaaagaag gtcaagtaac agtcatacag ctagaaaagt ccctgaaaaa 660
aagaattgtt aagaagtata ataacctttt caaaacccac aatgcagctt agttttcctt 720
tatttatttg tggtcatgaa gactatcccc atttctccat aaaatcctcc ctccatactg 780
ctgcattatg gcacaaaaga ctctaagtgc caccagacag aaggaccaga gtttctgatt 840
ataaacaatg atgctgggta atgtttaaat gagaacattg gatatggatg gtcagcccaa 900
cacaatggaa ttc 913

<210> 74
<211> 351
<212> DNA
<213> Homo sapien

<220>
<221> misc_feature
<222> (1)...(351)
<223> n = A,T,C or G

<400> 74
   tgtgcncagg ggatgggtgg gcngtggaga ngatgacaga aaggctggaa ggaanggggg 60
   tgggtttgaa ggccanggcc aaggggncct caggtccgnt tctgnnaagg gacagccttg 120
   aggaaggagn catggcaagc catagctagg ccaccaatca gattaagaaa nnctgagaaa 180
   nctagctgac catcactgtt ggtgnccagt ttcccaacac aatggaatnc caccacactg 240
   gactagngga nccactagtt ctagagcggc cgccaccgcg gtggaacccc aacttttgcc 300
   cctttagnga gggttaattg cgcgcttggc ntaatcatgg tcataagctg t 351
<210> 75
<211> 251
<212> DNA
<213> Homo sapien

<400> 75
tacttgacct tctttgaaaa gcattcccaa aatgctctat tttagataga ttaacattaa 60
ccaacataat tttttttaga tcgagtcagc ataaatttct aagtcagcct ctagtcgtgg 120
ttcatctctt tcacctgcat tttatttggt gtttgtctga agaaaggaaa gaggaaagca 180
aatacgaatt gtactatttg taccaaatct ttgggattca ttggcaaata atttcagtgt 240
ggtgtattat t 251

<210> 76
<211> 251
<212> DNA
<213> Homo sapien

<400> 76
tatttaataa tacaccacac tgaaattatt tgccaatgaa tcccaaagat ttggtacaaa 60
tagtacaatt cgtatttgct ttcctctttc ctttcttcag acaaacacca aataaaatgc 120
aggtgaaaga gatgaaccac gactagaggc tgacttagaa atttatgctg actcgatcta 180
aaaaaaatta tgttggttaa tgttaatcta tctaaaatag agcattttgg gaatgctttt 240
caaagaaggt c 251

<210> 77
<211> 351
<212> DNA
<213> Homo sapien

<220>
<221> misc_feature
<222> (1)...(351)
<223> n = A,T,C or G

<400> 77
actcaccgtg ctgtgtgctg tgtgcctgct gcctggcagc ctggccctgc cgctgctcag 60
gaggcgggag gcatgagtga gctacagtgg gaacaggctc aggactatct caagagannn 120
tatctctatg actcagaaac aaaaaatgcc aacagtttag aagccaaact caaggagatg 180
caaaaattct ttggcctacc tataactgga atgttaaact cccgcgtcat agaaataatg 240
cagaagccca gatgtggagt gccagatgtt gcagaatact cactatttcc aaatagccca 300
aaatggactt ccaaagtggt cacctacagg atcgtatcat atactcgaga c 351

<210> 78
<211> 1592
<212> DNA
<213> Homo sapien

<400> 78
gaattccatt gtgttggggc cctgggggcg gaggggaggg gcccaccacg gccttatttc 60
cgcgagcgcc ggcactgccc gctccgagcc cgtgtctgtc gggtgccgag ccaactttcc 120
tgcgtccatg cagccccgcc ggcaacggct gcccgctccc tggtccgggc ccaggggccc 180
gcgccccacc gccccgctgc tcgcgctgct gctgttgctc gccccggtgg cggcgcccgc 240
ggggtccggg gaccccgacg accctgggca gcctcaggat gctggggtcc cgcgcaggct 300
cctgcagcag gcggcgcgcg cggcgcttca cttcttcaac ttccggtccg gctcgcccag 360
cgcgctgcga gtgctggccg aggtgcagga gggccgcgcg tggattaatc caaaagaggg 420
atgtaaagtt cacgtggtct tcagcacaga gcgctacaac ccagagtctt tacttcagga 480
aggtgaggga cgtttgggga aatgttctgc tcgagtgttt ttcaagaatc agaaacccag 540
accaactatc aatgtaactt gtacacggct catcgagaaa aagaaaagac aacaagagga 600
ttacctgctt tacaagcaaa tgaagcaact gaaaaacccc ttggaaatag tcagcatacc 660
tgataatcat ggacatattg atccctctct gagactcatc tgggatttgg ctttccttgg 720
aagctcttac gtgatgtggg aaatgacaac acaggtgtca cactactact tggcacagct 780
cactagtgtg aggcagtgga aaactaatga tgatacaatt gattttgatt atactgttct 840
acttcatgaa ttatcaacac aggaaataat tccctgtcgc attcacttgg tctggtaccc 900
tggcaaacct cttaaagtga agtaccactg tcaagagcta cagacaccag aagaagcctc 960
cggaactgaa gaaggatcag ctgtagtacc aacagagctt agtaatttct aaaaagaaaa 1020
aatgatcttt ttccgacttc taaacaagtg actatactag cataaatcat tcttctagta 1080
aaacagctaa ggtatagaca ttctaataat ttgggaaaac ctatgattac aagtaaaaac 1140
tcagaaatgc aaagatgttg gttttttgtt tctcagtctg ctttagcttt taactctgga 1200
agcgcatgca cactgaactc tgctcagtgc taaacagtca ccagcaggtt cctcagggtt 1260
tcagccctaa aatgtaaaac ctggataatc agtgtatgtt gcaccagaat cagcattttt 1320
tttttaactg caaaaaatga tggtctcatc tctgaattta tatttctcat tcttttgaac 1380
atactatagc taatatattt tatgttgcta aattgcttct atctagcatg ttaaacaaag 1440
ataatatact ttcgatgaaa gtaaattata ggaaaaaaat taactgtttt aaaaagaact 1500
tgattatgtt ttatgatttc aggcaagtat tcatttttaa cttgctacct acttttaaat 1560
aaatgtttac atttctaaaa aaaaaaaaaa aa 1592

<210> 79
<211> 401
<212> DNA
<213> Homo sapien

<220>
<221> misc_feature
<222> (1)...(401)

<223> n = A,T,C or G

<400> 79
catactgtga attgttcttg actccttttc ttgacattca gttttcanaa tttccatctt 60
tcttctggaa ctaatgtgct gttctcttga ctgcctgctg ggccagcatc cgattgccag 120
ccagaaacgt cacactgccc aagatggcca ggtacttcaa ggtctggaac atgttgagct 180
gagtccagta gacatacatg agtcccagca tagcagcatg tcccaggtga aatataatcg 240
tgctaggagc aaaagtgaag ttggagacat tggcaccaat ccggatccac tagttctaga 300
gcggccgcca ccgcggtgga gctccagctt ttgttccctt tagtgagggt taattgcgcg 360
cttggcgtaa tcatggncat agctgtttcc tgtgtgaaat t 401

<210> 80
<211> 301
<212> DNA
<213> Homo sapien

<400> 80
aaaaatgaaa catctatttt agcagcaaga ggctgtgagg gatggggtag aaaaggcatc 60
ctgagagagt tctagaccga cccaggtcct gtggcacact atacgggtca ggaggggtgg 120
aagacaggcc taagctctag gacggtgaat ctcggggcta tttgtggatt tgttagaaac 180
agacattctt ttggcctttt cctggcactg gtgttgccgg caggtgggca gaagtgagcc 240
accagtcact gttcagtcat tgccaccaca gatcttcagc agaatcttcc ggtaatcccc 300
t 301

<210> 81
<211> 301
<212> DNA
<213> Homo sapien

<220>
<221> misc_feature
<222> (1)...(301)
<223> n = A,T,C or G

<400> 81
tagccaggtt gctcaagcta attttattct ttcccaacag gatccatttg gaaaatatca 60
agcctttaga atgtggcagc aagagaaagc ggactacgca ggaacgggga gtttgggaga 120
agctctcctg gtgttgactt agggatgaag gctccaggct gctgccagaa atggagtcac 180
cagcagaaga actgntttct ctgataagga tgtcccacca ttttcaagct gttcgttaaa 240
gttacacagg tccttcttgc agcagtaagt accgttagct cattttccct caagcgggtt 300
t 301

<210> 82
<211> 201
<212> DNA
<213> Homo sapien

<220>
<221> misc_feature
<222> (1)...(201)
<223> n = A,T,C or G

<400> 82
tcaacagaca aaaaaagttt attgaataca aaactcaaag gcatcaacag tcctgggccc 60
aagagatcca tggcaggaag tcaagagttc tgcttcaggg tcggtctggg cagccctgga 120
agaagtcatt gcacatgaca gtgatgagtg ccaggaaaac agcatactcc tggaaagtcc 180
acctgctggn cactgnttca t 201

<210> 83
<211> 251
<212> DNA
<213> Homo sapien

<220>
<221> misc_feature
<222> (1)...(251)
<223> n = A,T,C or G

<400> 83
gtaaggagca tactgtgccc atttattata gaatgcagtt aaaaaaaata ttttgaggtt 60
agcctctcca gtttaaaagc acttaacaag aaacacttgg acagcgatgc aatggtctct 120
cccaaaccgg ctccctctta ccaagtaccg taaacagggt ttgagaacgt tcaatcaatt 180
tcttgatatg aacaatcaaa gcatttaatg caaacatatt tgcttctcaa anaataaaac 240
cattttccaa a 251

<210> 84
<211> 301
<212> DNA
<213> Homo sapien

<220>
<221> misc_feature
<222> (1)...(301)
<223> n = A,T,C or G

<400> 84
agtttataat gttttactat gatttagggc ttttttttca aagaacaaaa attataagca 60
taaaaactca ggtatcagaa agactcaaaa ggctgttttt cactttgttc agattttgtt 120
tccaggcatt aagtgtgtca tacagttgtt gccactgctg ttttccaaat gtccgatgtg 180
tgctatgact gacaactact tttctctggg tctgatcaat tttgcagtan accattttag 240
ttcttacggc gtcnataaca aatgcttcaa catcatcagc tccaatctga agtcttgctg 300
c 301

<210> 85
<211> 201
<212> DNA
<213> Homo sapien

<400> 85
tatttgtgta tgtaacattt attgacatct acccactgca agtatagatg aataagacac 60
agtcacacca taaaggagtt tatccttaaa aggagtgaaa gacattcaaa aaccaactgc 120
aataaaaaag ggtgacataa ttgctaaatg gagtggagga acagtgctta tcaattcttg 180
attgggccac aatgatatac c 201

<210> 86
<211> 301
<212> DNA
<213> Homo sapien

<220>
<221> misc_feature
<222> (1)...(301)
<223> n = A,T,C or G

<400> 86
tttataaaat attttattta cagtagagct ttacaaaaat agtcttaaat taatacaaat 60
cccttttgca atataactta tatgactatc ttctcaaaaa cgtgacattc gattataaca 120
cataaactac atttatagtt gttaagtcac cttgtagtat aaatatgttt tcatcttttt 180
tttgtaataa ggtacatacc aataacaatg aacaatggac aacaaatctt attttgntat 240
tcttccaatg taaaattcat ctctggccaa aacaaaatta accaaagaaa agtaaaacaa 300
t 301

<210> 87
<211> 351
<212> DNA
<213> Homo sapien

<220>
<221> misc_feature
<222> (1)...(351)
<223> n = A,T,C or G

<400> 87
aaaaaagatt taagatcata aataggtcat tgttgtcaca acacatttca gaatcttaaa 60
aaaacaaaca ttttggcttt ctaagaaaaa gacttttaaa aaaaatcaat tccctcatca 120
ctgaaaggac ttgtacattt ttaaacttcc agtctcctaa ggcacagtat ttaatcagaa 180

tgccaatatt accaccctgc tgtagcanga ataaagaagc aagggattaa cacttaaaaa 240
aacngccaaa ttcctgaacc aaatcattgg cattttaaaa aagggataaa aaaacnggnt 300
aaggggggga gcattttaag taaagaangg ccaagggtgg tatgccngga c 351

<210> 88
<211> 301
<212> DNA
<213> Homo sapien

<220>
<221> misc_feature
<222> (1)...(301)
<223> n = A,T,C or G

<400> 88
gttttaggtc tttaccaatt tgattggttt atcaacaggg catgaggttt aaatatatct 60
ttgaggaaag gtaaagtcaa atttgacttc ataggtcatc ggcgtcctca ctcctgtgca 120
ttttctggtg gaagcacaca gttaattaac tcaagtgtgg cgntagcgat gctttttcat 180
ggngtcattt atccacttgg tgaacttgca cacttgaatg naaactcctg ggtcattggg 240
ntggccgcaa gggaaaggtc cccaagacac caaaccttgc agggtacctn tgcacaccaa 300
c 301

<210> 89
<211> 591
<212> DNA
<213> Homo sapien

<400> 89
tttttttttt tttttttatt aatcaaatga ttcaaaacaa ccatcattct gtcaatgccc 60
aagcacccag ctggtcctct ccccacatgt cacactctcc tcagcctctc ccccaaccct 120
gctctccctc ctcccctgcc ctagcccagg gacagagtct aggaggagcc tggggcagag 180
ctggaggcag gaagagagca ctggacagac agctatggtt tggattgggg aagagattag 240
gaagtaggtt cttaaagacc cttttttagt accagatatc cagccatatt cccagctcca 300
ttattcaaat catttcccat agcccagctc ctctctgttc tccccctact accaattctt 360
tggctcttac acaattttta tccctcaaat attcatccct ggcccaacca gtcccctgag 420
cctccctctg gtggagactc ctccacccat gagctcccca gagcatccaa gacagagtgc 480
acagagacct ggggaaggaa gctgaacttt gcagagatgt ggacaggtgc aggctagggt 540
acagggtggt ggtagaggag acaagtttta tttccaggcc cacagtctct c 591

<210> 90
<211> 1996
<212> DNA
<213> Homo sapien

<400> 90
tttttttttt ttttttatca aatgaatact ttattagaga cataacacgt ataaaataaa 60
tttcttttca tcatggagtt accagatttt aaaaccaacc aacactttct catttttaca 120
gctaagacat gttaaattct taaatgccat aatttttgtt caactgcttt gtcattcaac 180
tcacaagtct agaatgtgat taagctacaa atctaagtat tcacagatgt gtcttaggct 240
tggtttgtaa caatctagaa gcaatctgtt tacaaaagtg ccaccaaagc attttaaaga 300
aaccaattta atgccaccaa acataagcct gctatacctg ggaaacaaaa aatctcacac 360
ctaaattcta gcagagtaaa cgattccaac tagaatgtac tgtatatcca tatggcacat 420
ttatgacttt gtaatatgta attcataata caggtttagg tgtgtggtat ggagctagga 480
aaaccaaagt agtaggatat tatagaaaag atctgatgtt aagtataaag tcatatgcct 540
gatttcctca aaccttttgt ttttcctcat gtcttctgtc tttatatttt tatcacaaac 600
caagatctaa cagggttctt tctagaggat tattagataa gtaacacttg atcattaagc 660
acggatcatg ccactcattc atggttgttc tatgttccat gaactctaat agcccaactt 720
atacatggca ctccaagggg atgcttcagc cagaaagtaa agggctgaaa aagtagaaca 780
atacaaaagc cctcgtgtgg tgggaactgt ggcctcactc ttacttgtcc ttccattcaa 840
aacagtttgg cacctttcca tgacgaggat ctctacaggt aggttaaaat acttttctgt 900
gctattcagc cagaaatagt ttttgtgctg gatatgattt taaaacagat tttgtctgtc 960
accagtgcaa aaacattaca gatgtctggg ctaatacaaa aacacataag aatctacaac 1020
tttatattta atactctatt caaatttaac tcaaagtaat gcaaaataat tagaagtaaa 1080
aacttaattc ttctgagagc tctatttgga aaagcttcac atatccacac acaaatatgg 1140
gtatattcat gcacagggca aacaactgta ttctgaagca taaataaact caaagtaaga 1200
catcagtagc tagataccag ttccagtatt ggttaatggt ctctggggat cccattttaa 1260
gcactctcag atgaggatct tgctcagttg ttagactatc attagtttga ttaagcaact 1320
gaagtttact tcataaatta ctttttccta tatccaggac tctgcctgag aaattttata 1380

cattcctcca aaggtaagta ttctccaaag gtaagtattt gactattaac acaaaggcaa 1440
tgtgattatt gcataatgac actaaatatt atgtggcttt tctgttaggt ttataagttt 1500
tcaatgatca gttcaagaaa atgcagatca tatataacta aggttttaca ccagtggttg 1560
acaaactatg gcccacaggc taaacccagc ctccccttgt ttttataaat aagttttatt 1620
agacataacc acactcattc atttctgtat tgtgtatagc tgctttcacg ctatactagc 1680
agaactgaat agttgtgaca gagactgtat ggaccgtgaa gcataaatat ttaccatctg 1740
gcccattcta aaaaaagtgt gccaattcct ggtttacact aaaatataga gtttagtggg 1800
aagcctattt gaaatgtgtt tttttagggg gctgtaatta ccaattaaaa ttaaggttca 1860
ggtgactcag caaccaaaca aaagggatac taatttttta tgaacaatat atttgtattt 1920
tatggacata aaaggaaact ttcagaaaga aaaggaggaa aataaagggg gaaagggacc 1980
caacacaatg gaattc 1996

<210> 91
<211> 911
<212> DNA
<213> Homo sapien

<400> 91
gccctttttt tttttttttt cttgtttaaa aaaattgttt tcattttaat gatctgagtt 60
agtaacaaac aaatgtacaa aattgtcttt cacatttcca tacattgtgt tatggaccaa 120
atgaaaacgc tggactacaa atgcaggttt ctttatatcc ttaacttcaa ttattgtcac 180
ttataaataa aggtgatttg ctaacacatg catttgtgaa cacagatgcc aaaaattata 240
catgtaagtt aatgcacaac caagagtata cactgttcat ttgtgcagtt atgcgtcaaa 300
tgcgactgac acagaagcag ttatcctggg atatttcact ctatatgaaa agcatcttgg 360
agaaatagat tgaaatacag tttaaaacaa aaattgtatt ctacaaatac aataaaattt 420
gcaacttgca catctgaagc aacatttgag aaagctgctt caataaccct gctgttatat 480
tggttttata ggtatatctc caaagtcatg ggttgggata tagctgcttt aaagaaaata 540
aatatgtata ttaaaaggaa aatcacactt taaaaatgtg aggaaagctt tgaaaacagt 600
cttaatgcat gagtccatct acatattttc aagttttgga aacagaaaga agtttagaat 660
tttcaaagta atctgaaaac tttctaagcc attttaaaat aagatttttt tccccatctt 720
tccaatgttt cctatttgat agtgtaatac agaaatgggc agtttctagt gtcaacttaa 780
ctgtgctaat tcataagtca ttatacattt atgacttaag agttcaaata agtggaaatt 840
gggttataat gaaaatgaca agggggcccc ttcagcagcc actcatctga actagtaatc 900
ccaacacaat g 911

<210> 92
<211> 1710
<212> DNA
<213> Homo sapien

<400> 92
tttttttttt tttttaactt ttagcagtgt ttatttttgt taaaagaaac caattgaatt 60
gaaggtcaag acaccttctg attgcacaga ttaaacaaga aagtattact tatttcaact 120
ttacaaagca tcttattgat ttaaaaagat ccatactatt gataaagttc accatgaaca 180
tatatgtaat aaggagacta aaatattcat tttacatatc tacaacatgt atttcatatt 240
tctaatcaac cacaaatcat ataggaaaat atttaggtcc atgaaaaagt ttcaaaacat 300
taaaaaatta aagttttgaa acaaatcaca tgtgaaagct cattaaataa taacattgac 360
aaataaatag ttaatcagct ttacttatta gctgctgcca tgcatttctg gcattccatt 420
ccaagcgagg gtcagcatgc agggtataat ttcatactat gcgaccgtaa agagctacag 480
ggcttatttt tgaagtgaaa tgtcacaggg tctttcattc tctttcaaag gaagatcact 540
catggctgct aaactgttcc catgaagagt accaaaaaag cacctttctg aaatgttact 600
gtgaagattc atgacaacat atttttttta acctgttttg aaggagtttt gtttaggaga 660
ggggatgggc cagtagatgg agggtatctg agaagccctt ttctgtttta aaatataatg 720
attcactgat gtttatagta tcaacagtct tttaagaaca atgaggaatt aaaactacag 780
gatacgtgga atttaaatgc aaattgcatt catggatata cctacatctt gaaaaacttg 840
aaaaggaaaa actattccca aagaaggtcc tgatacttaa gacagcttgc tgggtttgat 900
caaagcagaa agcatatact ttcaagtgag aaaacagcag tggcaggctt gagtcttcca 960
agcaatcaaa tctgtaaagc agatggttac tagtaagtct agttatggga gtctgagttc 1020
taactcatgc tgtgcttgct ggatttgctg gctcttttcc gctctctgtg atgctggact 1080
ggcttgtcag gtgacatgct ctcaaagttg tgactggact cgttgtgctg ccgggtgtac 1140
ctcttgcact tgcaggcagt gactactgtg atttgtagg tgcgtgtgct gccatcttgg 1200
cactgcagct ggattctctg ggtacgggtt ttgtcattga cacaccgcca ctcctgggag 1260
ctcctcctgc tccagtactt tgttccatag cctcctccaa tccagttagg gagcactggc 1320
aggggcaagc actcgccagc acacaccagc tccttcagag ggctgatgct ggtgcactgg 1380
ccatcagaga tgtatttggt ggaacgcagt tcccggcaac ccacttgaac ccgagtgttc 1440
cgatccagtc cagtgttact gaaatgcctg cctccatttc tggcttgatt caacgtgctg 1500
ttgctgctgg ggtgtgctgg aacaggttta accacatgtg aataaaggat ttctgtggca 1560
tcatttttaa aagccaaaca gcttttcatt aggatgcatg caaggggaag gagatagaaa 1620

tgaatggcag gaggaagcat ggtgagtaga ggatttgctt gactgaagag ctggttaatt 1680
cttttgcctc tgcccaacac aatggaattc 1710

<210> 93
<211> 251
<212> DNA
<213> Homo sapien

<400> 93
cccaccctac ccaaatatta gacaccaaca cagaaaagct agcaatggat tcccttctac 60
tttgttaaat aaataagtta aatatttaaa tgcctgtgtc tctgtgatgg caacagaagg 120
accaacaggc cacatcctga taaaaggtaa gaggggggtg gatcagcaaa aagacagtgc 180
tgtgggctga ggggacctgg ttcttgtgtg ttgcccctca agactcttcc cctacaaata 240
actttcatat g 251

<210> 94
<211> 738
<212> DNA
<213> Homo sapien

<400> 94
cccttttttt ttttttttcc acttctcagt ttatttctgg gactaaattt gggtcagagc 60
tgcagagaag ggatgggccc tgagcttgag gatgaaagtg ccccagggag attgagacgc 120
aacccccgcc ctggacagtt ttggaaattg ttcccagggt tcaactagag agacacggtc 180
agcccaatgt gggggaagca gaccctgagt ccaggagaca tggggtcagg ggctggagag 240
atgaacattc tcaacatctc tgggaaggaa tgagggtctg aaaggagtgt cagggctgtc 300
cctgcagcag gtggggatgc cggtgtgctg agtcctggga tgactcagga gttggcctgg 360
atggtttcct ggatccactt ggtgaacttg cagaggttcg tgtagacacc cggtctgttg 420
ggccgggcac aagggtaatc tccccaggac acgagtccct gcagggagcc attgcagacc 480
acaggccccc cagaatcacc ctggcaggag tctctacctg ctttgtcacc ggcgcagaac 540
atggtgtcat ctatctgtct cgggtaagca tcctcgcacc ttttctgact tagcacgctg 600
atattcaagc actggaggac cttagggaag tgcacttggg ggctcttggt tgtcccccag 660
ccagacacca agcactttgt cccagcagag ggacaatgag aggagacgtt gatgggtctg 720
acatctttag tgggacga 738